# EUROPEAN PATENT SPECIFICATION

(11) **EP 4 247 814 B1**
(45) Date of publication and mention of the grant of the patent: **01.01.2025**
(21) Application number: 21814770.0
(22) Date of filing: 18.11.2021
(51) Int. Cl.: C07D 487/04, A61K 31/519, A61P 35/00

(54) **CRYSTALLINE FORMS OF 4-AMINO-N-[4-(METHOXYMETHYL)PHENYL]-7-(1-METHYLCYCLOPROPYL)-6-(3-MORPHOLINOPROP-1-YN-1-YL)-7H-PYRROLO[2,3-D]PYRIMIDINE-5-CARBOXAMIDE, METHODS OF PREPARATION, AND USES THEREOF**
KRISTALLINE FORMEN VON 4-AMINO-N-[4-(METHOXYMETHYL)PHENYL)-7-(1-METHYLCYCLOPROPYL)-6-(3-MORPHOLINOPROP-1-YN-1-YL)-7H-PYRROLO[2,3-D!PYRIMIDIN-5-CARBOXAMID, HERSTELLUNGSVERFAHREN UND VERWENDUNGEN DAVON
FORMES CRISTALLINES DE 4-AMINO-N-[4-(MÉTHOXYMÉTHYLE))PHÉNYL]-7-(1-MÉTHYLCYCLOPROPYLE)-6-(3-MORPHOLINOPROP-1-YN-1-YL)-7 H-PYRROLO [2,3-D] PYRIMIDINE-5-CARBOXAMIDE, LEURS PROCÉDÉS DE PRÉPARATION ET LEURS UTILISATIONS

(30) Priority: 20.11.2020 US 202063116191 P
(43) Date of publication of application: 27.09.2023
(73) Proprietor: Taiho Pharmaceutical Co., Ltd., Chiyoda-ku Tokyo 101-8444 (JP)
(72) Inventor: FRASCA, Gionata, 6512 Giubiasco (CH); FUMAGALLI, Tiziano, 22030 Lipomo (CO) (IT); GIAFFREDA, Stefano Luca, 40026 Imola (BO) (IT); MODENA, Enrico, 40138 Bologna (IT); IANNI, Cristina, 40138 Bologna (IT)
(74) Representative: M. Zardi & Co S.A.
(86) International application number: PCT/EP2021/082093
(87) International publication number: WO 2022/106514

(56) References cited:
- US-B2- 10 155 768

## Description

### FIELD

The present disclosure relates to crystalline forms of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide (also referred to as HM06 or TAS953) a selective RET inhibitor useful in the treatment of cancer. The present disclosure also relates to crystalline forms of HM06, both free base and salt forms, and methods of producing the same.

### BACKGROUND

Various protein kinases are present in vivo, and are known to be involved in a wide range of functional regulations. RET is a receptor tyrosine kinase identified as one of the protooncogenes. RET binds to the glial cell line-derived neurotrophic factor (GDNF) and GDNF receptor to form a complex, which enables RET to perform physiological functions through intracellular phosphorylation signaling. (Bavetsias et al., "Aurora Kinase Inhibitors: Current Status and Outlook", Frontiers in Oncology, 2015, vol. 5, Art.278.) Some studies indicate that in cancers, such as lung cancer, thyroid cancer, breast cancer, pancreas cancer, and prostate cancer, the translocation, mutation, and overexpression of RET enhances its activation to thereby contribute to cell growth, tumor formation, or tissue infiltration. (Kohno et al., "KIF5B-RET fusions in lung adenocarcinoma," Nature Med., 18(3): pp. 375-377, (2012); Santoro et al., "RET/PTC activation in papillary thyroid carcinoma: European Journal of Endocrinology Prize Lecture," Eur J Endocrinol., 155: pp. 645-653, (2006); Yeganeh et al., "RET Prato Oncogene Mutation Detection and Medullary Thyroid Carcinoma Prevention," Asian Pac J Cancer Prev., 16(6): pp. 2107-2117, (2015); Gattelli et al., "Ret inhibition decreases growth and metastatic potential of estrogen receptor positive breast cancer cells," EMBO Mol Med., 5: pp. 1335-1350, (2013); Ito et a., "Expression of glial cell line-derived neurotrophic factor family members and their receptors in pancreatic cancers," Surgery, 138: pp. 788-794, (2005); and Dawson et al., "Altered Expression of RET Proto-oncogene Product in Prostatic Intraepithelial Neoplasia and Prostate Cancer," J Natl Cancer Inst., 90: pp. 519-523, (1998)). In addition, RET is known to be a poor prognostic factor of cancer, as indicated in some reports that the translocation of RET and its enhanced activation level are also inversely correlated with prognosis in cancer (Cai et al., "KIF5B-RET Fusions in Chinese Patients With Non-Small Cell Lung Cancer," Cancer, 119: pp. 1486-1494, (2013); Elisei et al., "Prognostic Significance of Somatic RET Oncogene Mutations in Sporadic Medullary Thyroid Cancer: A 10-Year Follow-Up Study,"J Clin Endocrinol Metab., 93(3): pp. 682-687, (2008); Gattelli et al., "Ret inhibition decreases growth and metastatic potential of estrogen receptor positive breast cancer cells," EMBO Mol Med., 5: pp. 1335-1350, (2013); and Zeng et al., "The Relationship between Over-expression of Glial Cell-derived Neurotrophic Factor and Its RET Receptor with Progression and Prognosis of Human Pancreatic Cancer," J. Int. Med. Res., 36: pp. 656-664, (2008)). Therefore, an inhibitor capable of inhibiting RET activity is thought to be useful as a therapeutic agent for diseases associated with abnormally enhanced RET signaling pathways, including cancers.

Furthermore, many cancers can lead to a metastatic brain tumor. Symptomatic metastatic brain tumors have been reported to occur in 8 to 10% of cancer patients, and there is also a report that, in lung cancer, brain metastasis has been reported at a frequency of 40 to 50% according to autopsy. (Qingbei Zeng, J Med Chem. 22; 58(20): 8200-15, (2015); Lakshmi Nayak, Curr Oncol Rep; 14(1): 48-54, (2012); Brunilde Gril, Eur J Cancer.; 46(7): 1204-10, (2010)). Accordingly, it is desirable to find treatments that effectively treat cancer, including brain metastasis of the cancer.

Even more desirable is that the treatment can be administered in a form that is easily absorbed by the body and also shelf stable. The pharmaceutically active substance used to prepare the treatment should be as pure as possible and its stability on long-term storage should be guaranteed under various environmental conditions. These properties are useful to prevent the appearance of unintended degradation products in pharmaceutical compositions, which degradation products may be potentially toxic or result simply in reducing the potency of the composition.

A primary concern for the large-scale manufacture of pharmaceutical compounds is that the active substance should have a stable crystalline morphology to ensure consistent processing parameters and pharmaceutical quality. If an unstable crystalline form is used, crystal morphology may change during manufacture and/or storage resulting in quality control problems and formulation irregularities. Such a change may affect the reproducibility of the manufacturing process and thus lead to final formulations which do not meet the high quality and stringent requirements imposed on formulations of pharmaceutical compositions. In this regard, it should be generally borne in mind that any change to the solid state of a pharmaceutical composition which can improve its physical and chemical stability gives a significant advantage over less stable forms of the same drug.

When a compound crystallizes from a solution or slurry, it may crystallize with different spatial lattice arrangements, a property referred to as "polymorphism." Each of the crystal forms is a "polymorph." While polymorphs of a given substance have the same chemical composition, they may differ from each other with respect to one or more physical properties, such as solubility, dissociation, true density, dissolution, melting point, crystal shape, compaction behavior, flow properties, and/or solid state stability.

RET inhibitor 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide (also known as HM06 or TAS0953) is reported in U.S. Patent No. 10,155,768. The molecular formula of the free base form of HM06/TAS0953 is C₂₆H₃₀N₆O₃, the molecular weight is 474.57, and the structural formula of the free base is:

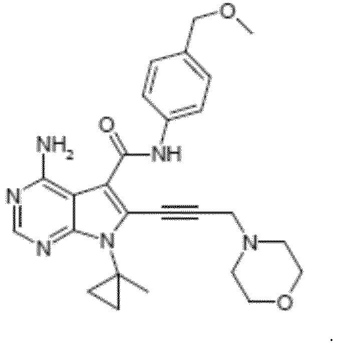

However, crystalline forms of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide have not been heretofore disclosed.

### BRIEF SUMMARY

Accordingly, disclosed herein are substantially crystalline forms of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base, HCl salt forms, processes for making said crystalline forms, and methods for using said forms.

The present disclosure relates to substantially crystalline forms of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide. In one aspect of the present disclosure, the crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide is a free base. In one aspect of the present disclosure, the crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide is an HCl salt, for example a 1:1 or 1:2 HCl salt.

The present disclosure also relates to a pharmaceutical composition comprising at least one substantially crystalline form as described herein and a pharmaceutically acceptable excipient.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present disclosure further relates to a method of treating cancer in a human patient in need thereof comprising administering to the patient an effective amount of a substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide.

Additional objects and advantages will be set forth in part in the description which follows, and in part will be obvious from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

The accompanying drawings, which are incorporated in and constitute a part of this specification, illustrate one (several) embodiment(s) and together with the description, serve to explain the principles described herein.

### BRIEF DESCRIPTION OF THE DRAWINGS

**Figure 1A** shows an XRPD pattern of HM06 crystalline free base Form 1 obtained using CuKα radiation.
**Figure 1B** shows an XRPD pattern of HM06 crystalline free base Form 2 obtained using CuKα radiation.
**Figure 1C** shows an XRPD pattern of HM06 crystalline free base Form 3 obtained using CuKα radiation.
**Figure 1D** shows an XRPD pattern of HM06 crystalline free base Form 4 obtained using CuKα radiation.
**Figure 1E** shows an XRPD pattern of HM06 crystalline free base Form 5 obtained using CuKα radiation.
**Figure 1F** shows an XRPD pattern of an HM06 HCl crystalline salt Form A obtained using CuKα radiation.
**Figure 1G** shows an overlay of XRPD patterns of five HM06 crystalline free base forms and an HM06 HCl crystalline salt Form A obtained using CuKα radiation.
**Figure 2A** shows an XRPD pattern of crystalline HM06 1:1 HCl Form 1 obtained using CuKα radiation.
**Figure 2B** shows an XRPD pattern of crystalline HM06 1:1 HCl Form 1 obtained using CuKα radiation.
**Figure 2C** shows an XRPD pattern of crystalline HM06 1:1 HCl Form 1 obtained using CuKα radiation.
**Figure 2D** is a DSC thermogram of HM06 1:1 HCl Form 1
**Figure 2E** provides the TGA profile of HM06 1:1 HCl Form 1.
**Figure 3A** shows an XRPD pattern the crystalline HM06 1:2 HCl Form 1 obtained using CuKα radiation.
**Figure 3B** provides the TGA profile of HM06 1:2 HCl Form 1.
**Figure 3C** is a DSC thermogram of HM06 1:2 HCl crystalline Form 1.

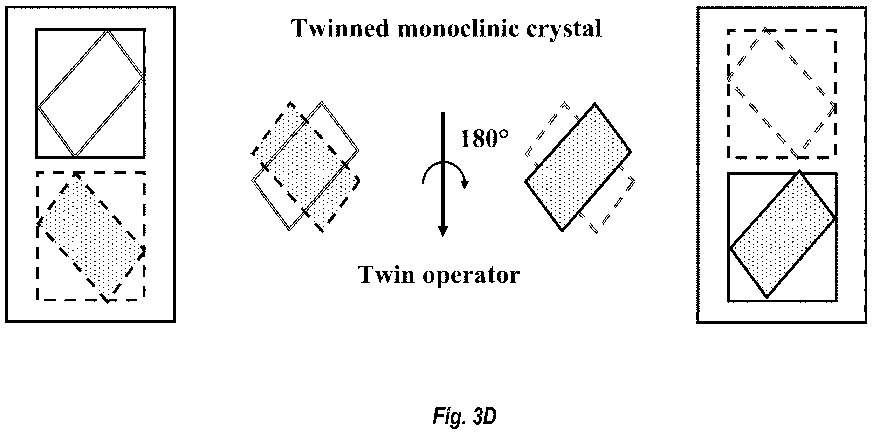

**Figure 3D** illustrates crystal twinning through space-group transition from orthorhombic to monoclinic. A macroscopic crystal is shown from the bottom and top view. A twinned monoclinic crystal results if the molecules rearrange in the same manner in A as viewed from the top and in B as viewed from the bottom. The monoclinic packing is equivalent in the two volumes but is not related by crystallographic symmetry; instead, the twin operator, a 180° rotation around an axis in the plane perpendicular to the unique crystallographic b axis of the monoclinic lattice, relates the diffraction patterns of the two volumes.
**Figure 3E** provides an ORTEP drawing of the single crystal of HM06 1:2 HCl Form 1.

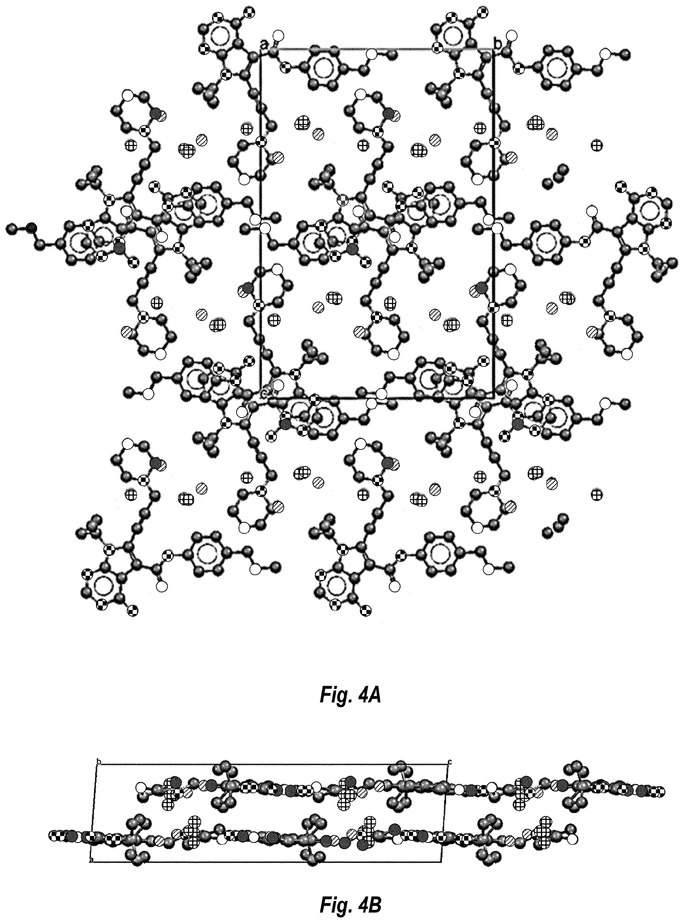

**Figure 4A** illustrates crystal packing view of HM06 1:2 HCl Form 1 along the "a axis."
**Figure 4B** illustrates crystal packing view of HM06 1:2 HCl Form 1 along the "b axis."
**Figure 5A** shows an XRPD pattern of crystalline HM06 1:2 HCl Form 1-bis obtained using CuKα radiation.
**Figure 5B** shows an XRPD pattern of HM06 1:2 HCl crystalline Form 1-bis obtained using CuKα radiation.
**Figure 5C** is an overlay of XRPD pattern profiles in the range of 2θ = 25.5° to 27.5°of HM06 1:2 HCl Form 1-bis under vacuum (time = zero) and in air from 2 to 20 minutes.
**Figure 5D** shows an overlay of XRPD pattern profiles for HM06 1:2 HCl Form 1-bis as starting material, under vacuum, and after uptake of water from the air.
**Figure 6A** shows an XRPD pattern overly of the HM06 1:2 HCl Form 2 sample obtained after 50°C slurry experiments from ethanol (top pattern). Standard reference patterns of Form 1 and Form 2 (bottom two patterns) are reported for comparison.
**Figure 6B** shows an XRPD pattern overly of the HM06 1:2 HCl Form 2 sample obtained after 50°C slurry experiments from ethanol (top 2 patterns). Standard reference patterns of Form 2 (bottom pattern) and Form 3 (second from bottom pattern) are reported for comparison.
**Figure 6C** shows the XRPD pattern of HM06 1:2 HCl Form 2 collected after 4-days slurry experiments from ethanol scaled on 100 mg and used as STD reference obtained using CuKα radiation.
**Figure 6D** is a DSC thermogram of HM06 1:2 HCl Form 2.
**Figure 6E** provides the TGA profile of HM06 1:2 HCl Form 2.
**Figure 6F** provides XRPD patterns of samples collected after micro scale up R01 (second from top pattern) and R02 (top pattern) of Example 5. Standard reference XRPD patterns for Forms 2 and 3 provided as the bottom two patterns are included for comparison.
**Figure 6G** provides an XRPD pattern of a samples collected after a micro scale up procedure using a concentration of 20 mg/mL (top line). Standard reference XRPD patterns for Forms 2 and 3 provided as the bottom two lines are included for comparison.
**Figure 7A** shows an XRPD pattern overlay of crystalline HM06 1:2 HCl Form 3 obtained after 50°C slurry experiments from Acetonitrile (blue line), the standard reference patterns of Form 1 (black line), Form 2 (green line), and Form 3 (pink line) obtained using CuKα radiation.
**Figure 7B** shows an XRPD pattern of HM06 1:2 HCl Form 3 collected after fast gradient precipitation from 1-Propanol scaled on 100 mg obtained using CuKα radiation.
**Figure 7C** is a DSC thermogram of HM06 1:2 HCl Form 3
**Figure 7D** provides the TGA profile of HM06 1:2 HCl Form 3
**Figure 8A** shows an XRPD pattern of HM06 1:2 HCl Form 4-bis obtained using using CuKα radiation (top pattern) with an XRPD pattern of HM06 1:2 HCl Form 1 for reference (bottom pattern)
**Figure 8B** shows an XRPD pattern of HM06 1:2 HCl Form 4-bis.
**Figure 8C** shows an XRPD patterns overlay of HM06 1:2 HCl Form 4-bis (bottom pattern) and the same sample analyzed after 7 days storage in a sealed vial (middle pattern). An XRPD pattern for HM06 1:2 HCl Form 1 is provided for comparison (top pattern).
**Figure 8D** shows an XRPD pattern of HM06 1:2 HCl Form 4 obtained using CuKα radiation.
**Figure 8E** shows an XRPD patterns overlay of HM06 1:2 HCl Form 4 (bottom pattern) and after storage overnight at 43% relative humidity (top pattern).
**Figure 9A** shows the XRPD pattern of HM06 1:2 HCl Form 5-bis (top pattern) compared with Form 5 (bottom pattern).
**Figure 9B** shows an XRPD pattern of HM06 1:2 HCl Form 5-bis obtained using CuKα radiation.
**Figure 9C** shows an XRPD patterns overlay of HM06 1:2 HCl Form 5-bis (blue top pattern) and the same sample analyzed after 18 hours of exposure (bottom red pattern).
**Figure 9D** shows XRPD patterns overlay of HM06 1:2 HCl Form 5-bis (blue top pattern) and the same sample analyzed after 7 days in a sealed vial (middle pattern). An XRPD pattern for HM06 1:2 HCl Form 1 is provided for comparison (bottom pattern).
**Figure 9E** shows the XRPD pattern of HM06 1:2 HCl Form 5 obtained using CuKα radiation.
**Figure 9F** is a DSC thermogram of HM06 1:2 HCl Form 5.
**Figure 9G** provides the TGA profile of HM06 1:2 HCl Form 5.
**Figure 10** shows an XRPD pattern of HM06 1:2 HCl Form 6.
**Figure 11** shows an overlay of XRPD patterns of the isolated forms of crystalline HM06 1:2 HCl.

### DESCRIPTION OF THE EMBODIMENTS

As summarized above, and as set forth in detail below, the present disclosure relates to crystalline forms of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide (also referred to as HM06 or TAS953). The present disclosure also relates to methods of making the crystalline free base forms and HCl salt forms thereof, such as a dichloride (or 1:2) HCl salt:

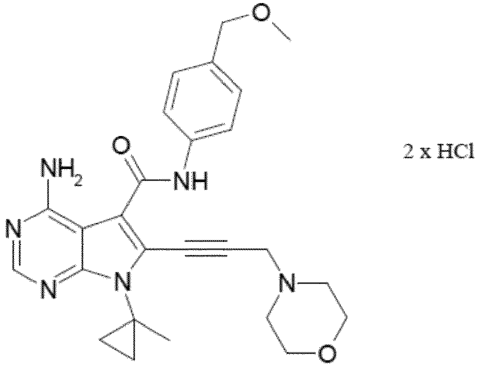

Also disclosed herein are methods of using the crystalline forms for therapeutic treatment such as for cancer.

The details of the disclosure are set forth in the accompanying description below. Although methods and materials similar or equivalent to those described herein can be used in the practice or testing of the present disclosure, illustrative methods and materials are now described. Other features, objects, and advantages of the disclosure will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this disclosure belongs. All patents and publications cited in this specification are incorporated herein by reference in their entireties.

The present disclosure relates to substantially crystalline forms of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide. In at least one aspect of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide is a free base.

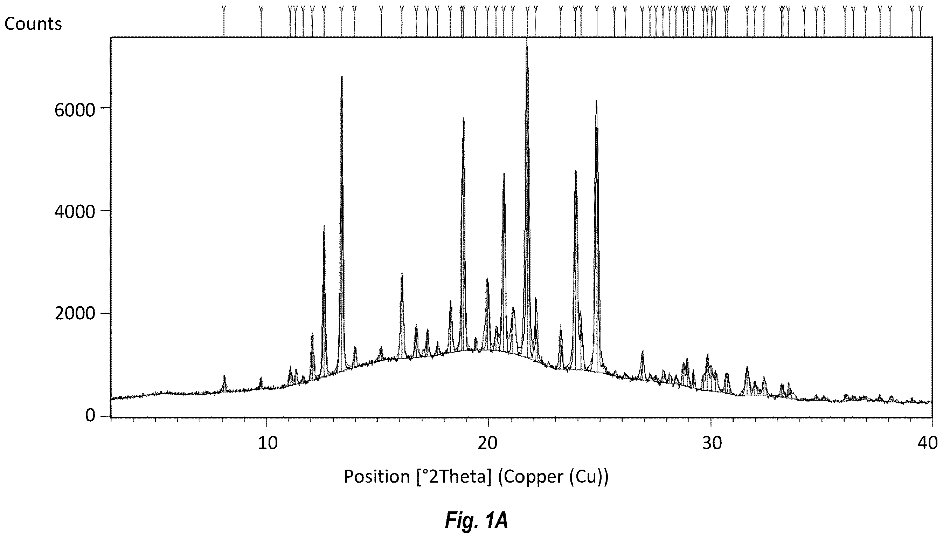

In some embodiments of the present disclosure the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base is Form 1. In at least one embodiment the 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 1 is characterized by an XRPD pattern substantially the same as Figure 1A. In at least one embodiment the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 1 is characterized by an XRPD pattern comprising one or more peaks chosen from peaks at about 12.57°2θ, 13.36°2θ, 16.08°2θ, 18.86°2θ, 20.66°2θ, 21.73°2θ, 23.90°2θ, and 24.86°2θ.

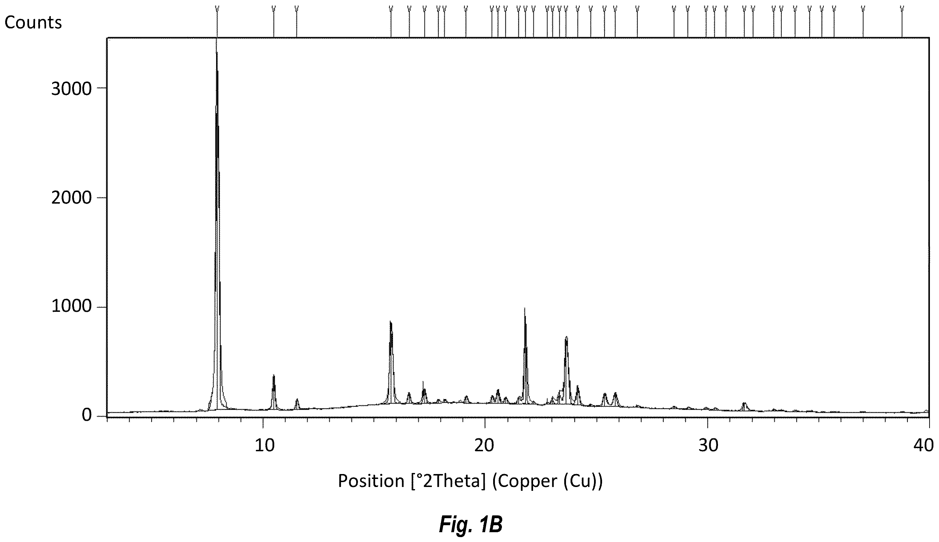

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base is Form 2. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 2 is characterized by an XRPD pattern substantially the same as Figure 1B. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 2 is characterized by an XRPD pattern comprising one or more peaks chosen from peaks at about 7.94°2θ, 10.47°2θ, 11.53°2θ, 15.75°2θ, 21.80°2θ, and 23.65°2θ.

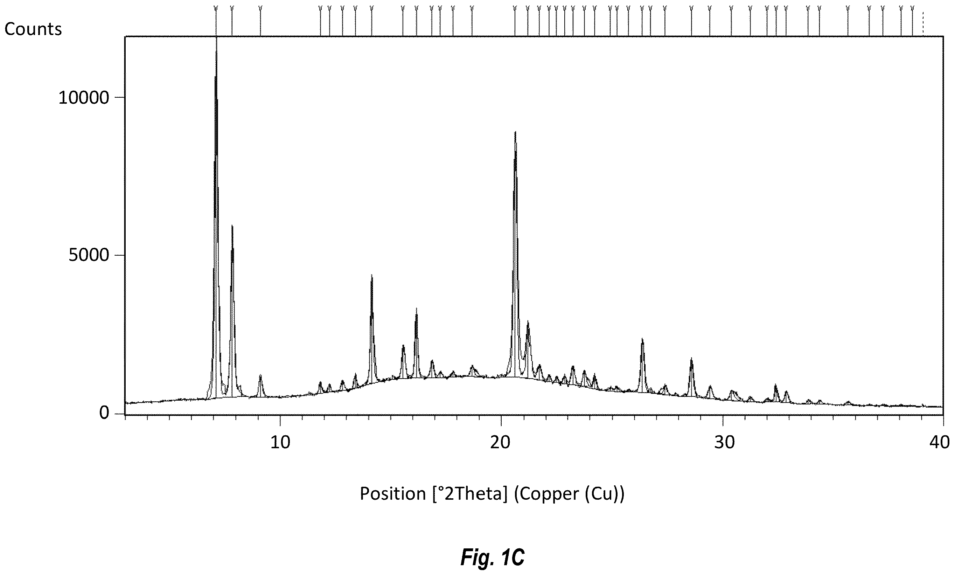

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base is Form 3. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 3 is characterized by an XRPD pattern substantially the same as Figure 1C. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 3 is characterized by an XRPD pattern comprising one or more peaks chosen from peaks at about 7.11°2θ, 7.83°2θ, 14.12°2θ, 16.15°2θ, 20.61°2θ, 21.19°2θ, 26.37°2θ, and 28.59°2θ.

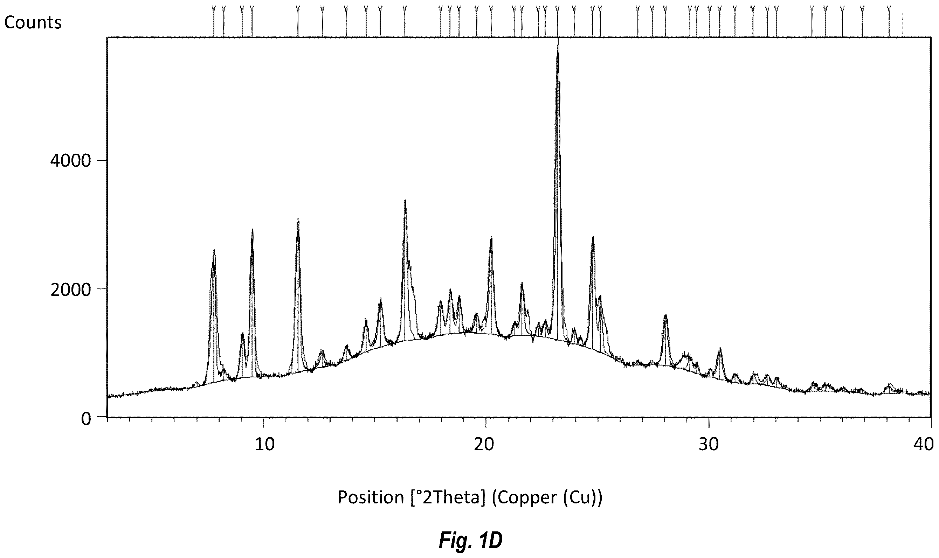

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base is Form 4. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 4 is characterized by an XRPD pattern substantially the same as Figure 1D. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 4 is characterized by an XRPD pattern comprising one or more peaks chosen from peaks at about 7.77°2θ, 9.48°2θ, 11.54°2θ, 16.34°2θ, 20.21°2θ, 23.24°2θ, and 24.77°2θ.

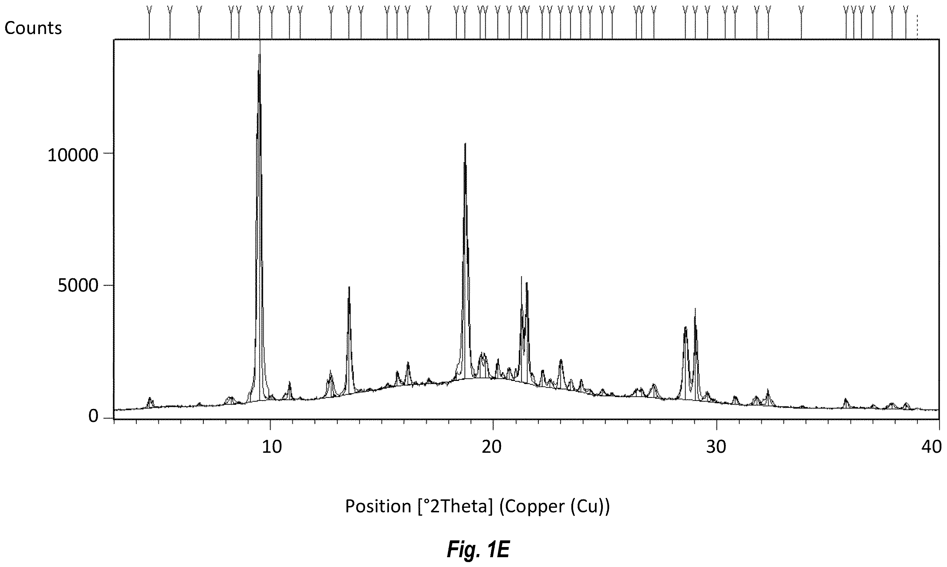

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base is Form 5. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 5 is characterized by an XRPD pattern substantially the same as Figure 1E. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 5 is characterized by an XRPD pattern comprising one or more peaks chosen from peaks at about 9.51°2θ, 13.52°2θ, 18.71°2θ, 21.26°2θ, 21.49°2θ, 28.60°2θ, and 29.05°2θ.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide is a mixture of free base forms.

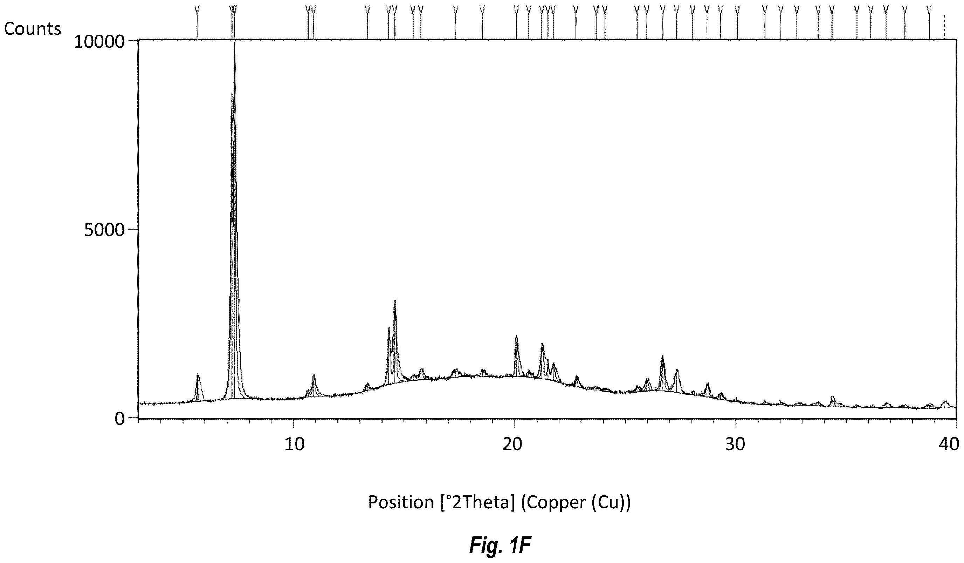

In some embodiments, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide is an HCl salt Form A. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide HCl salt Form A is characterized by an XRPD pattern substantially the same as Figure 1F. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide HCl salt Form A is characterized by an XRPD pattern comprising one or more peaks chosen from peaks at about 5.67°2θ, 7.19°2θ, 7.32°2θ, 10.90°2θ, 14.31°2θ, 14.59°2θ, 20.08°2θ, and 21.24°2θ.

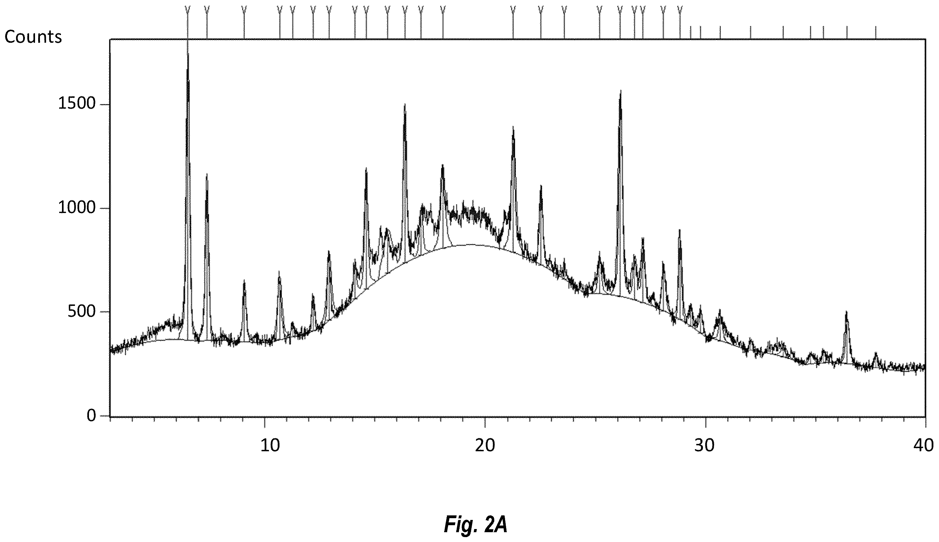

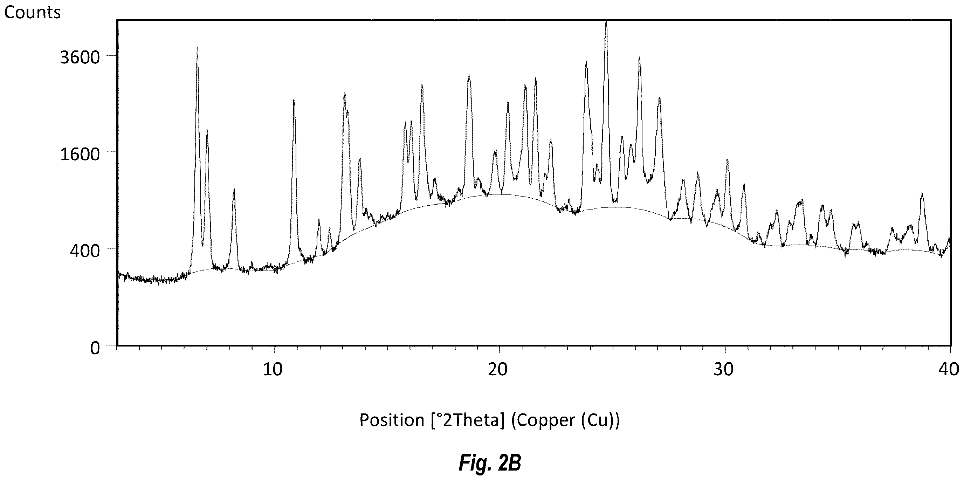

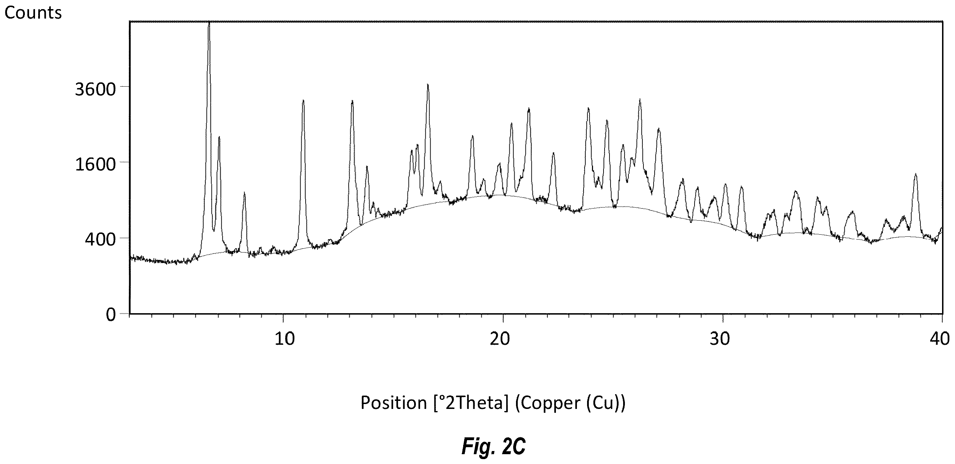

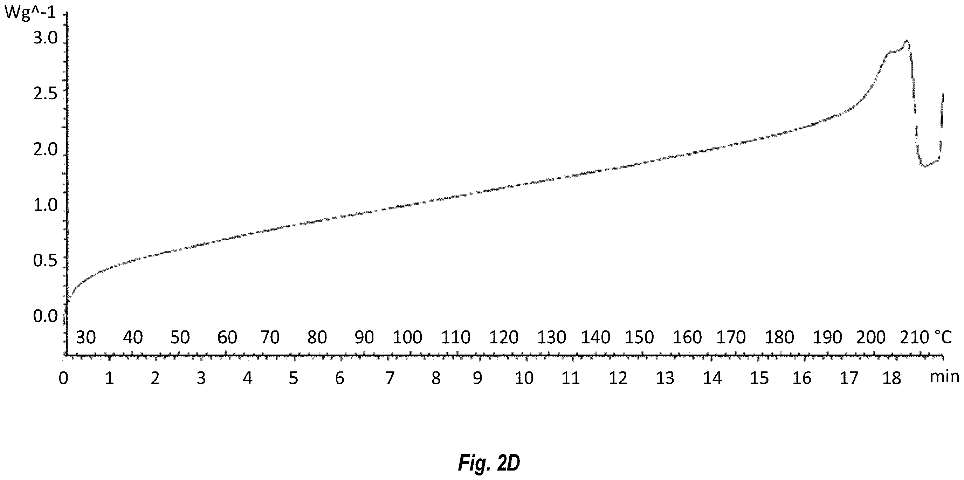

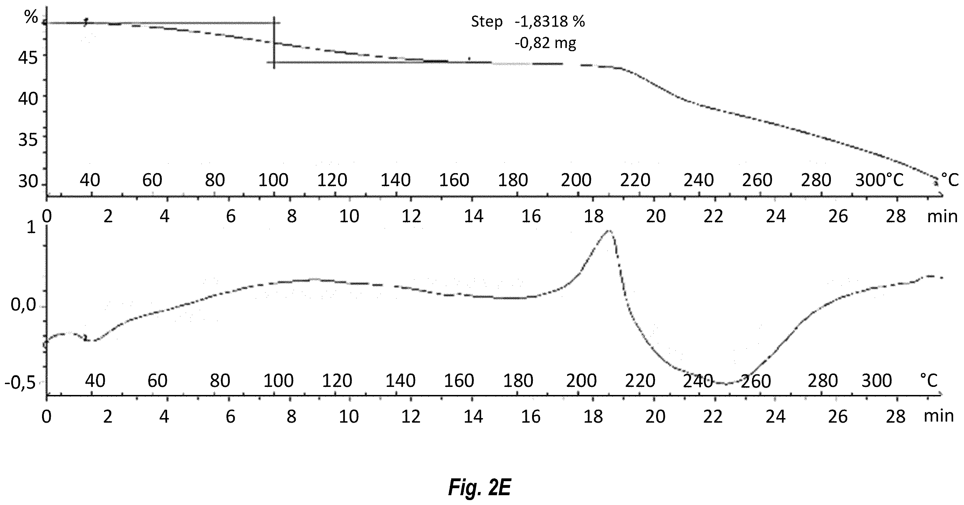

In some embodiments of the present disclosure, the substantially crystalline form is 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:1 HCl salt. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:1 HCl salt is Form 1. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:1 HCl salt Form 1 is characterized by an XRPD pattern substantially the same as Figure 2A, 2B, or 2C. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:1 HCl Form 1 is characterized by an XRPD pattern comprising one or more peaks chosen from peaks at about 6.53°2θ, 7.37°2θ, 9.07°2θ, 14.60°2θ, 16.35°2θ, 21.26°2θ, and 26.12°2θ. In at least one embodiment, the substantially crystalline form of 4-amino-N-[4-(methoxymethyl)phenyl]-7-(1-methylcyclopropyl)-6-(3 -morpholinoprop-1-yn-1-yl)-7H-pyrrolo[2,3-d]pyrimidine-5-carboxamide 1:1 HCl Form 1 has at least one characteristic chosen from a DSC thermogram substantially the same as Figure 2D and a TGA profile substantially the same as Figure 2E.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide is a mixture of HCl Form A and 1:1 HCl Form 1.

In some embodiments, the substantially crystalline form is 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt.

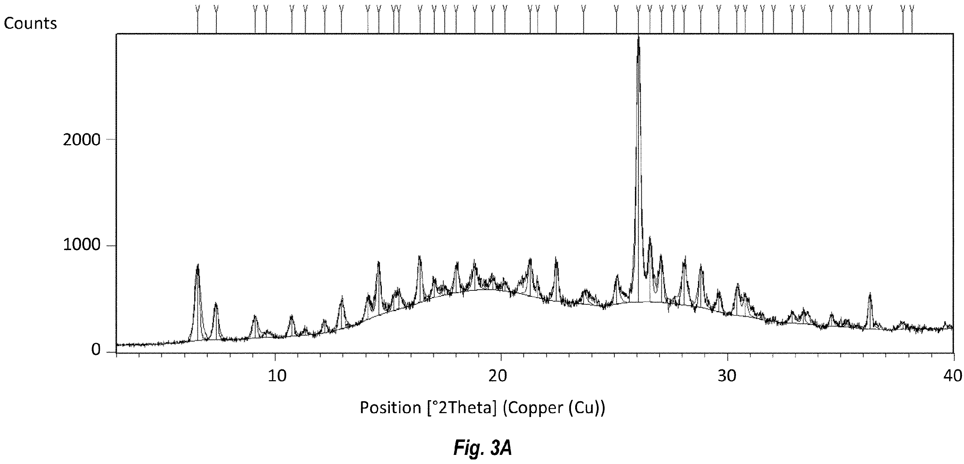

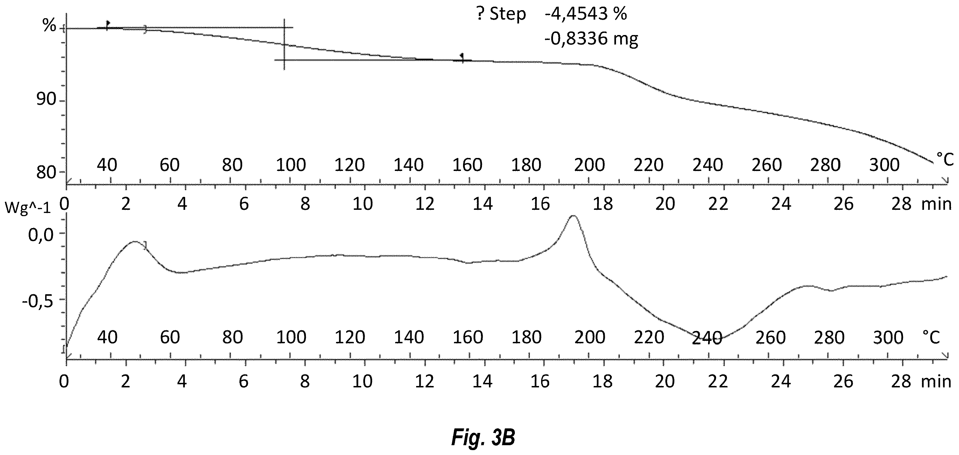

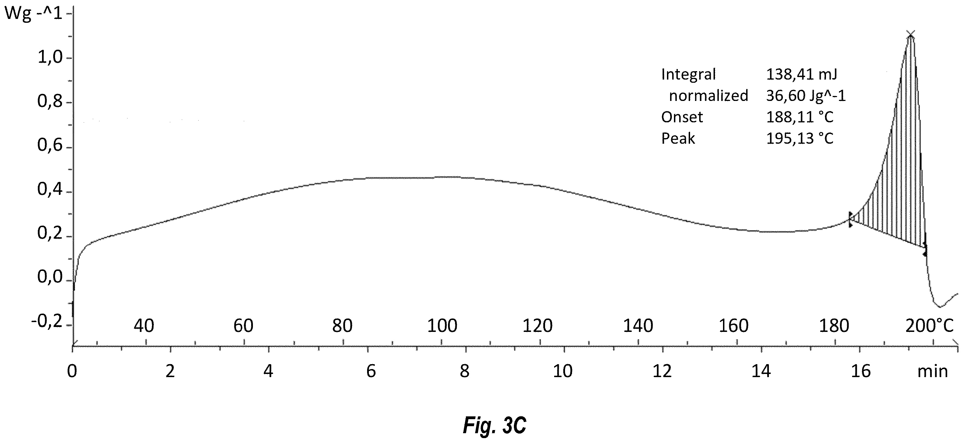

In some embodiments, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 1. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 1 is characterized by an XRPD pattern substantially the same as Figure 3A. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl Form 1 is characterized by an XRPD pattern comprising one or more peaks chosen from peaks at about 6.59°2θ, 7.40°2θ, 9.12°2θ, 14.57°2θ, 16.39°2θ, 26.06°2θ, 26.57°2θ, and 27.07°2θ. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl Form 1 has at least one characteristic chosen from a DSC thermogram substantially the same as Figure 3C and a TGA profile substantially the same as Figure 3B.

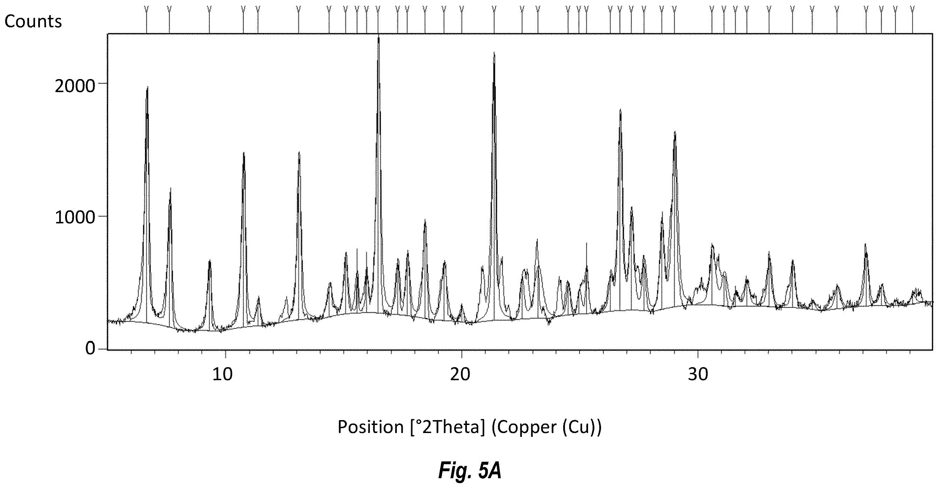

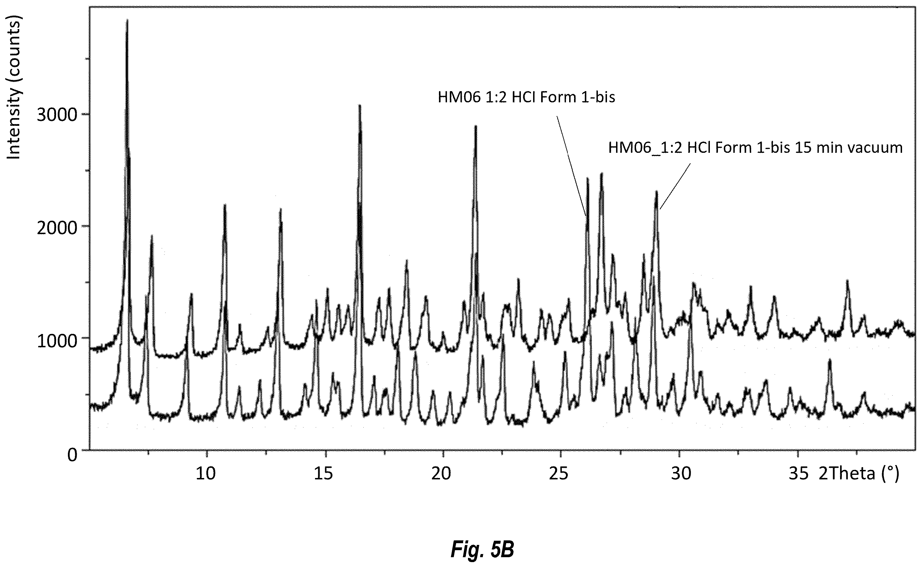

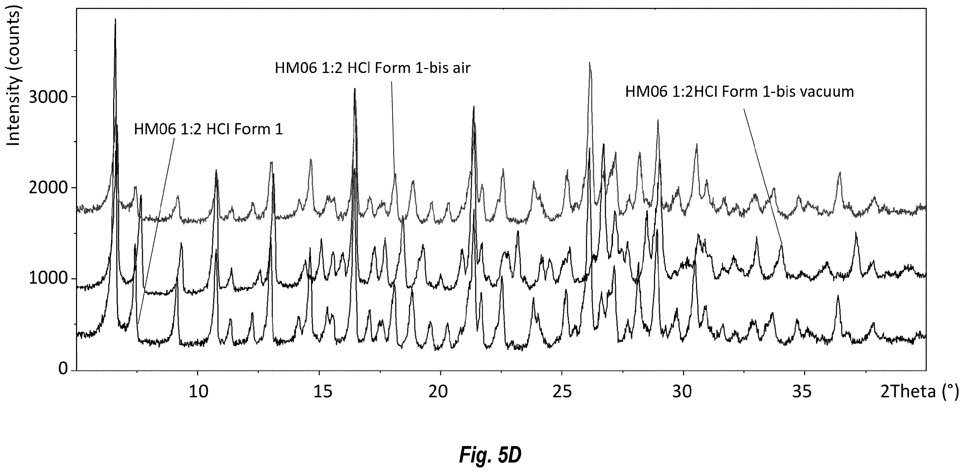

In some embodiments, the substantially crystalline form is 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 1-bis. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 1-bis is characterized by an XRPD pattern substantially the same as Figure 5A or Figure 5B. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 1-bis is characterized by an XRPD pattern comprising one or more peaks chosen from peaks at about 6.66°2θ, 7.63°2θ, 9.31°2θ, 10.74°2θ, 13.09°2θ, 16.45°2θ, 21.36°2θ, 26.70°2θ, and 29.01°2θ.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of Form 1 and Form 1-bis.

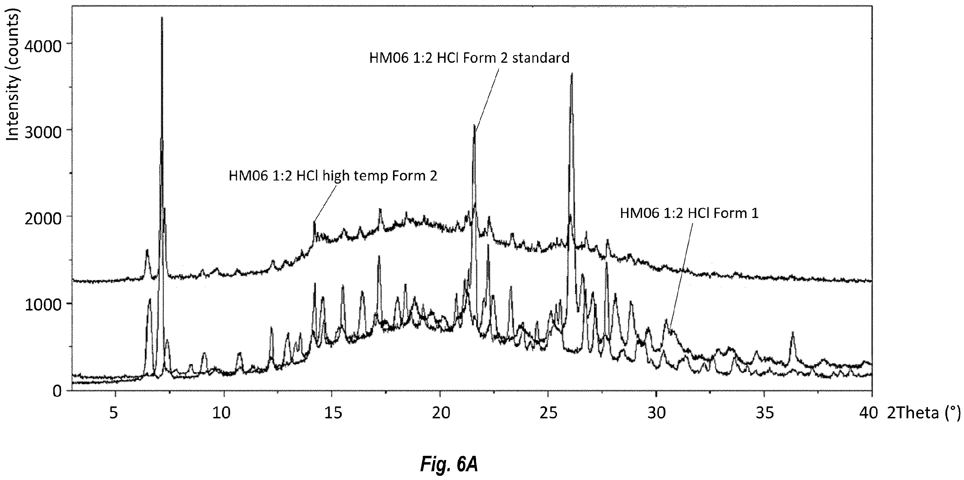

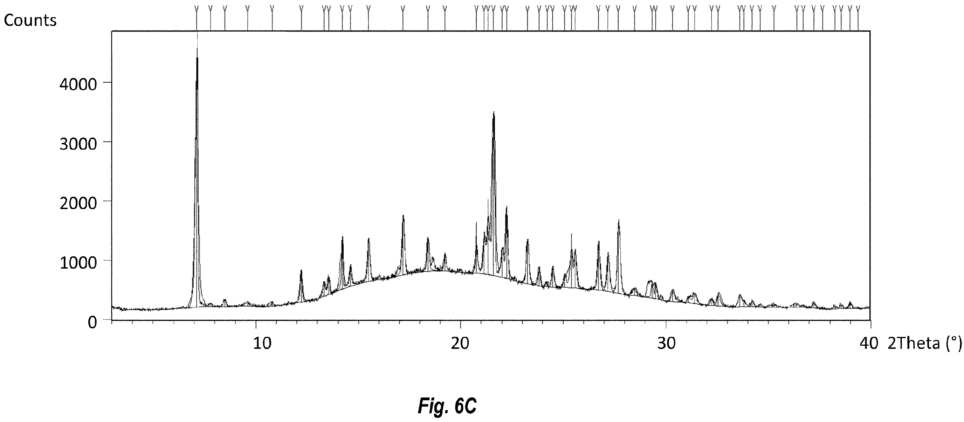

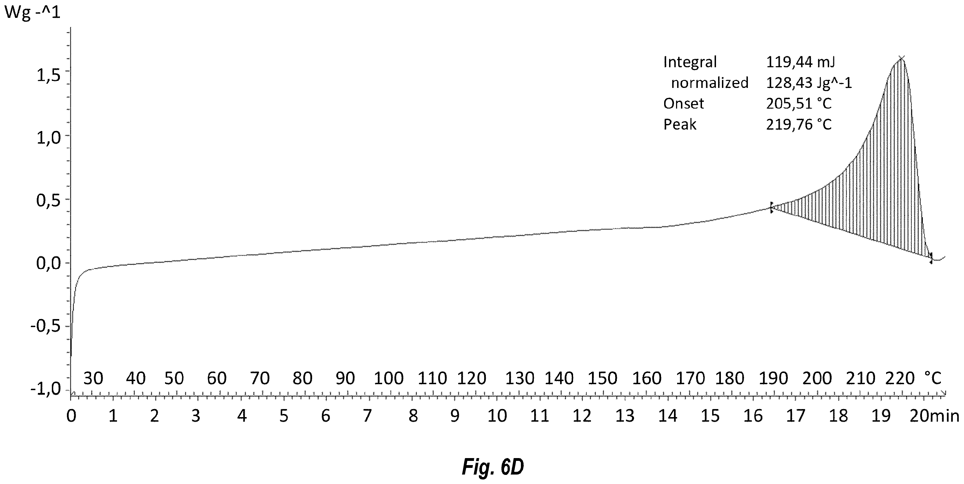

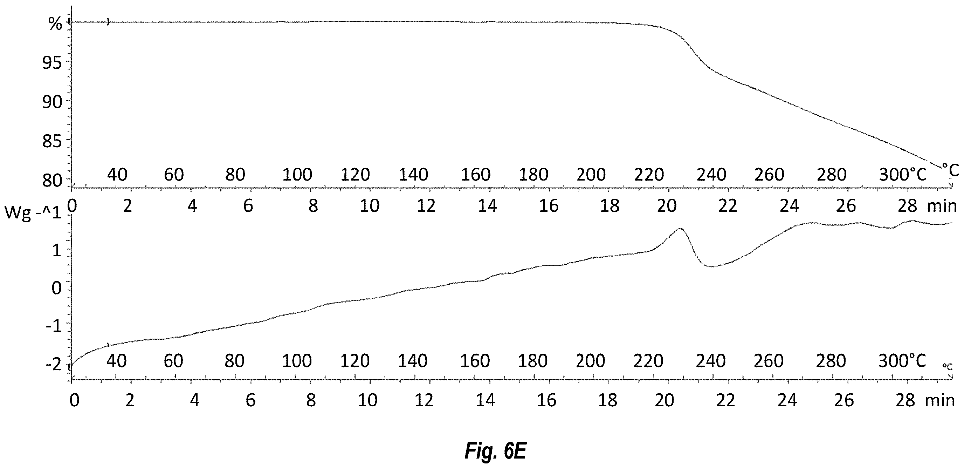

In some embodiments of the present disclosure, the substantially crystalline form 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 2. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 2 is characterized by an XRPD pattern substantially the same as Figure 6C. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 2 is characterized by an XRPD pattern comprising one or more peaks chosen from peaks at about 7.13°2θ, 12.21°2θ, 14.22°2θ, 15.50°2θ, 17.18°2θ, 21.60°2θ, 22.23°2θ, 23.26°2θ, 26.72°2θ, and 27.69°2θ. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 2 has at least one characteristic chosen from a DSC thermogram substantially the same as Figure 6D and a TGA profile substantially the same as Figure 6E.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of Form 1 and Form 2.

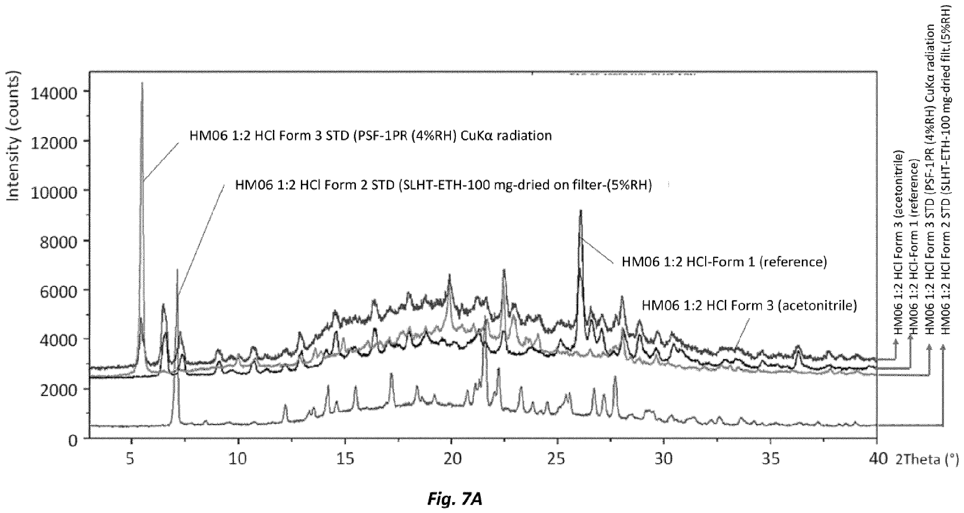

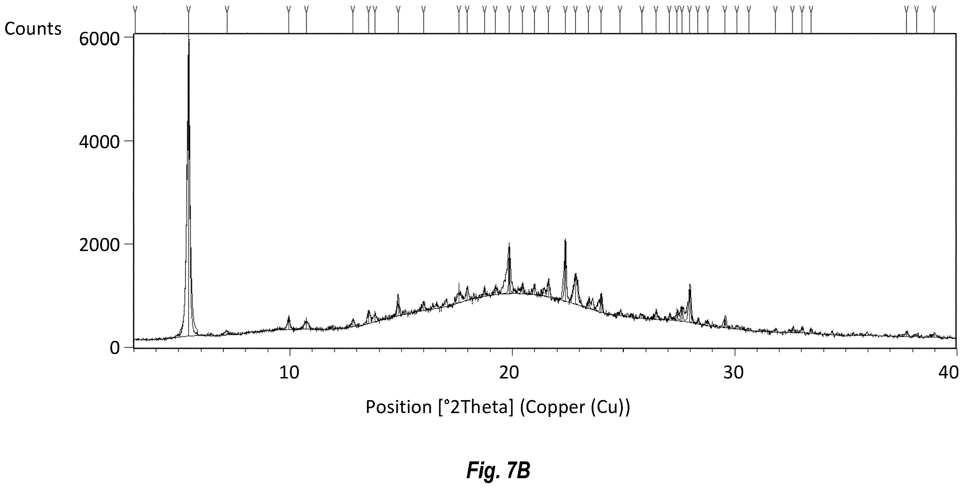

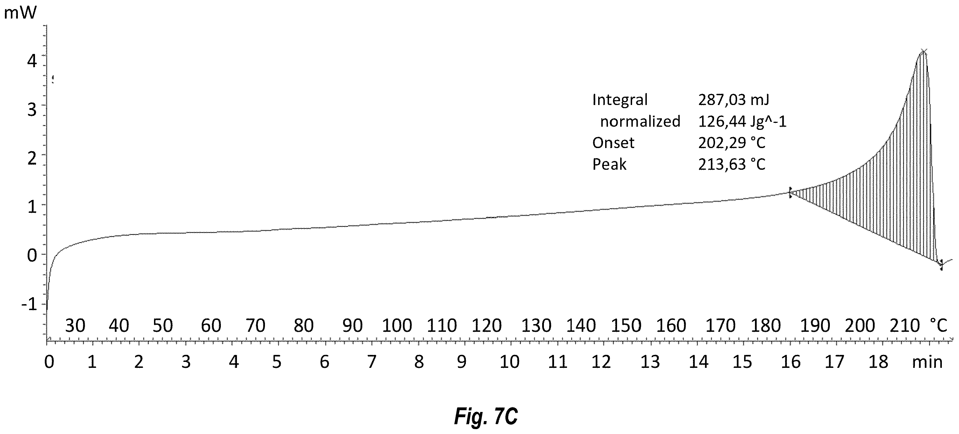

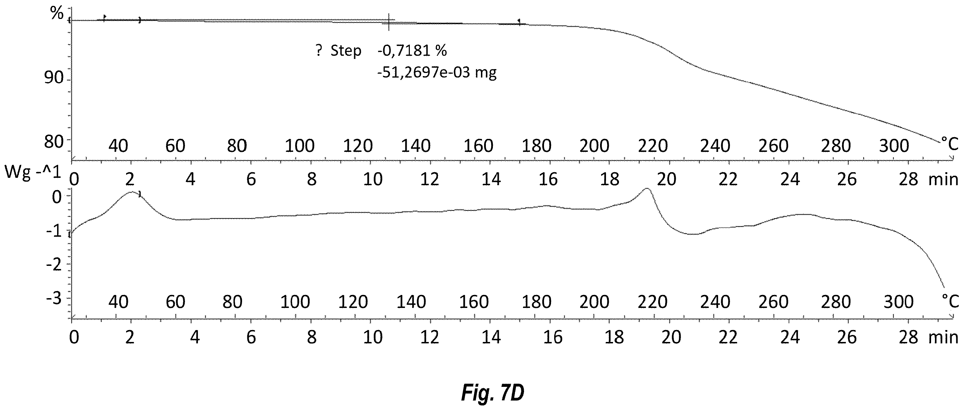

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 3. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 3 is characterized by an XRPD pattern substantially the same as Figure 7B. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 3 is characterized by an XRPD pattern comprising one or more peaks chosen from peaks at about 5.44°2θ, 9.94°2θ, 14.85°2θ, 22.39°2θ, 22.84°2θ, and 27.96°2θ. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 3 has at least one characteristic chosen from a DSC thermogram substantially the same as Figure 7C and a TGA profile substantially the same as Figure 7D.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of Form 2 and Form 3.

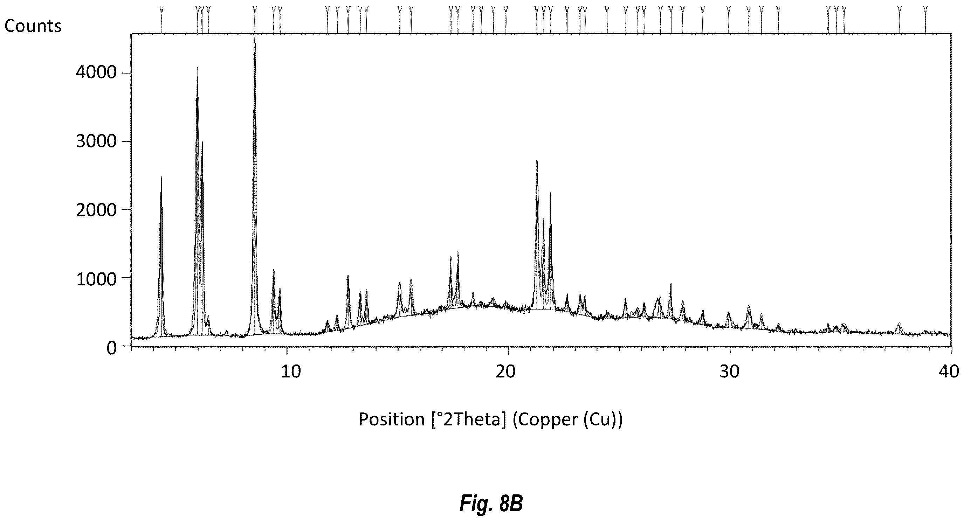

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 4-bis. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 4-bis is characterized by an XRPD pattern substantially the same as Figure 8B. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 4-bis is characterized by an XRPD pattern comprising one or more peaks chosen from peaks at about 4.35°2θ, 5.98°2θ, 6.20°2θ, 8.54°2θ, 17.39°2θ, 21.28°2θ, 21.58, and 21.89°2θ.

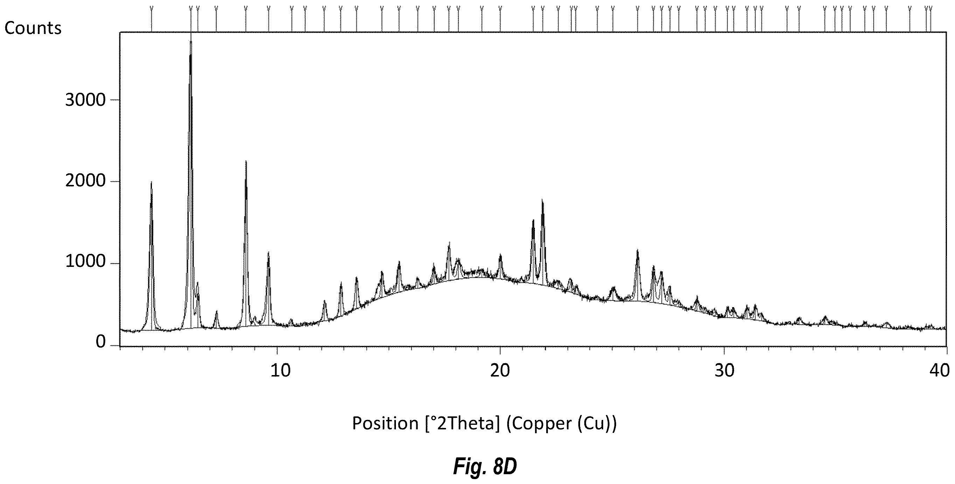

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 4. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 4 is characterized by an XRPD pattern substantially the same as Figure 8D. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 4 is characterized by an XRPD pattern comprising one or more peaks chosen from peaks at about 4.38°2θ. 6.15°2θ, 8.60°2θ, 9.62°2θ, 21.46°2θ, 21.90°2θ, and 26.14°2θ.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of Form 4-bis and Form 4.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of Form 1 and Form 4.

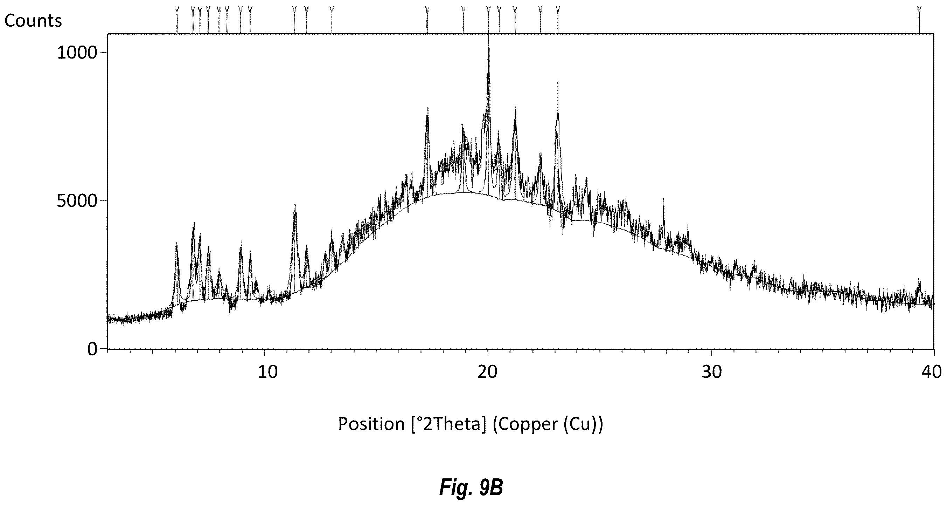

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 5-bis. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 5-bis is characterized by an XRPD pattern substantially the same as Figure 9B. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 5-bis is characterized by an XRPD pattern comprising one or more peaks chosen from peaks at about 6.08°2θ, 6.82°2θ, 7.11°2θ, 7.51°2θ, 8.92°2θ, 9.35°2θ, 11.34°2θ, 17.29°2θ, 20.02°2θ, 21.21°2θ, 22.36°2θ, and 23.15°2θ.

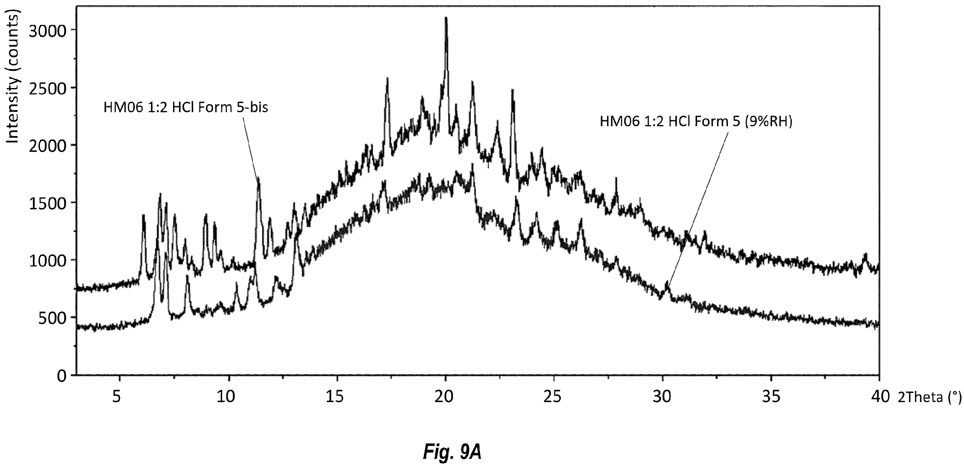

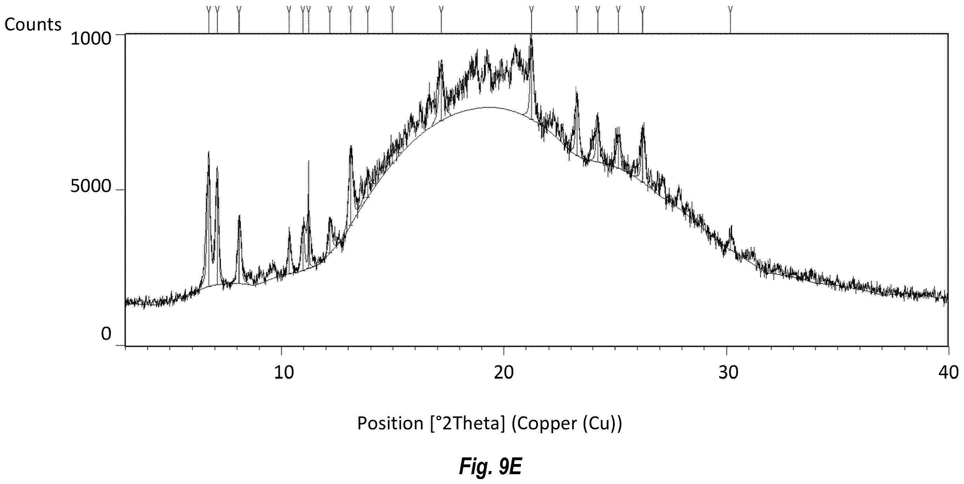

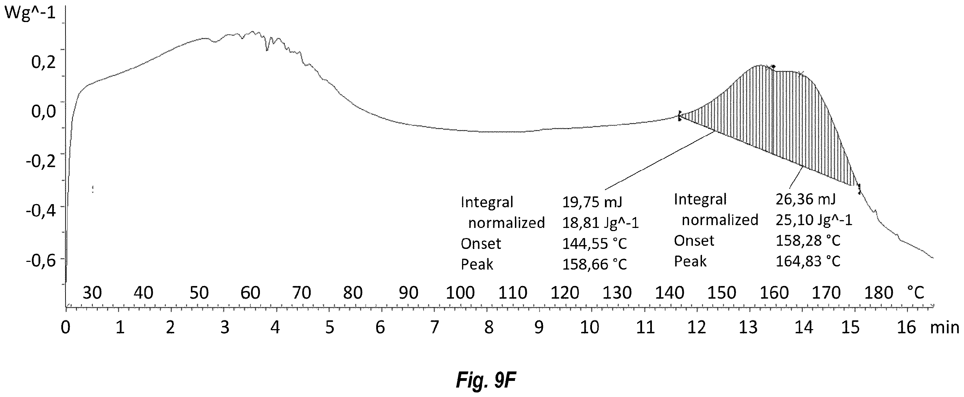

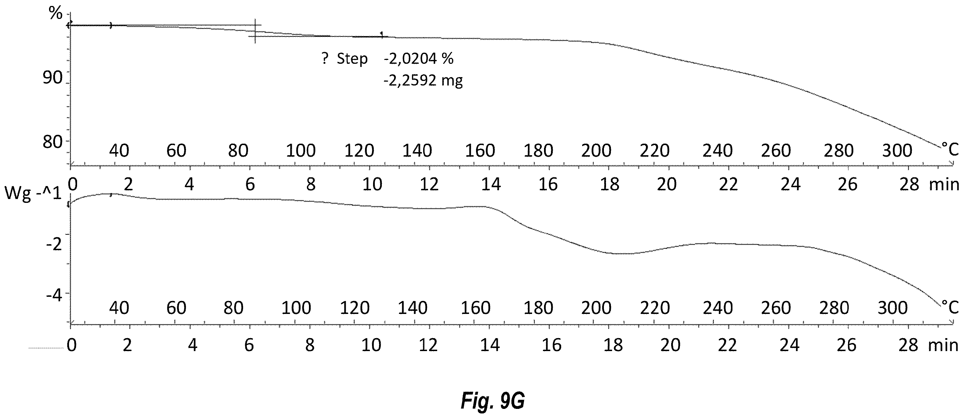

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 5. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 5 is characterized by an XRPD pattern substantially the same as Figure 9E. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 5-bis is characterized by an XRPD pattern comprising one or more peaks chosen from peaks at about 6.74°2θ, 7.11°2θ, 8.10°2θ, 13.10°2θ, 17.16°2θ, 23.28°2θ, 24.22°2θ, 25.15°2θ, and 26.24°2θ. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 5 has at least one characteristic chosen from a DSC thermogram substantially the same as Figure 9F and a TGA profile substantially the same as Figure 9G.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of Form 5-bis and Form 5.

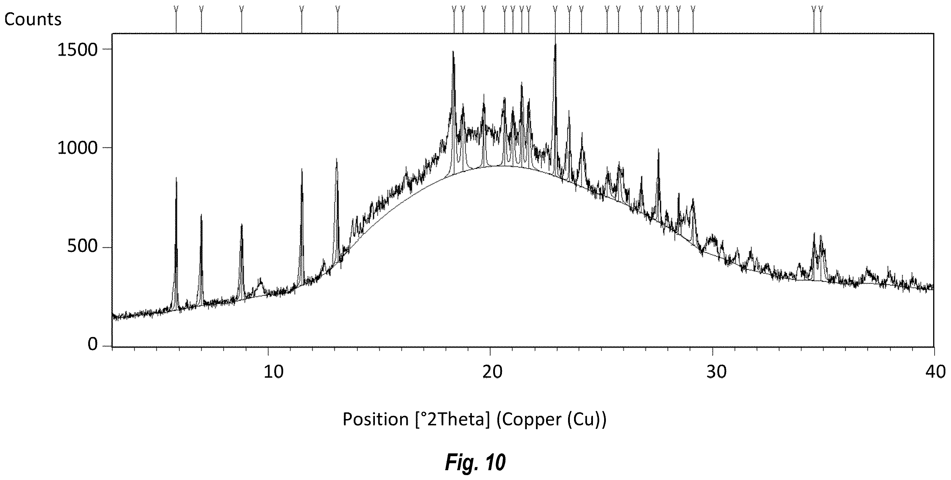

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is Form 6. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 6 is characterized by an XRPD pattern substantially the same as Figure 10. In at least one embodiment, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt Form 6 is characterized by an XRPD pattern comprising one or more peaks chosen from peaks at about 5.88°2θ, 7.01°2θ, 8.81°2θ, 11.51°2θ, 13.12°2θ, 18.36°2θ, 21.4°2θ, and 22.92°2θ.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of Form 1, Form 2, and Form 3.

In some embodiments of the present disclosure, the substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt is a mixture of at least one form chosen from Form 1, Form 1-bis, Form 2, Form 3, Form 4-bis, Form 4, Form 5-bis, Form 5, and Form 6.

The substantially crystalline forms disclosed herein can be in at least 50% crystalline form, such as at least 60%, at least 70%, at least 80%, at least 90%, at least 95%, at least 96%, at least 97%, at least 98%, at least 99%, or 100% crystalline.

The present disclosure also relates to pharmaceutical compositions comprising at least one substantially crystalline form as disclosed herein and a pharmaceutically acceptable excipient. For example, in some embodiments, the pharmaceutical compositions can comprise the substantially crystalline forms of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt.

The present disclosure still further relates to a method of treating cancer in a human patient in need thereof comprising administering to the patient an effective amount of a substantially crystalline form of 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide. In at least one embodiment, the substantially crystalline form is 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide free base Form 1. In at least one embodiment, the substantially crystalline form is 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:1 HCl Form 1. In at least one embodiment, the substantially crystalline form is 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl Form 1.

**Table 1. Abbreviations and names used in the following examples and elsewhere herein include:**

| **Abbreviation** | **Definition** |
|---|---|
| atm | atmospheres |
| ACN | acetonitrile |
| DCM | dichloromethane |
| DSC | Differential Scanning Calorimetry |
| eq. | equivalents |
| EtOH or ETH | ethanol |
| EVLT | Evaporation experiment at low temperature |
| EVRT | Evaporation experiment at room temperature |
| FS | Freely soluble |
| Gr | Grinding |
| h | hours |
| H₂O | water |
| HCl | hydrochloric acid |
| HPLC | high pressure liquid chromatography |
| IPC | In-process control |
| min | minutes |
| PP | Polypropylene |
| PPT | Precipitate |
| PSF | Precipitation by temperature gradient fast (in ice/acetone bath) |
| PSS | Precipitation by temperature gradient slow (0.5°C/min) |
| PTFE | Polytetrafluoroethylene |
| RH | Relative Humidity |
| RT | Room temperature |
| S | Soluble |
| S at HT | Soluble at HT |
| SLBM_HT | Slurry experiment at high temperature in binary mixture |
| SLBM_RT | Slurry experiment at room temperature in binary mixture |
| SLRT-15D | Slurry experiment at room temperature for 15 days |
| SLRT-3D | Slurry experiment at room temperature for 3 days |
| SLHT | Slurry experiment at high temperature |
| SLVT | Slurry experiment at variable temperature |
| SM | Starting Material |
| SS | Sparingly soluble |
| STA | Simultaneous Thermal Analysis |
| THF | tetrahydrofuran |
| Ti | Internal Temperature |
| Tj | Jacket Temperature |
| VSS | Very slightly soluble |
| VT-XRPD | Variable temperature X-ray powder diffraction |
| VP-XRPD | Variable Pressure X-ray powder diffraction |
| SC-XRD | Single crystal X-ray diffraction |
| XRPD | X-ray powder diffraction |

### EXAMPLES

### EXPERIMENTAL AND INSTRUMENT DETAILS

Unless otherwise specified, following instruments and parameters were used for the physical characterization of the crystalline forms disclosed herein.

X-Ray Powder Diffraction (XRPD) Analysis

| | |
|---|---|
| Instrument type: | Rigaku MiniFlex600 |
| Application SW: | Miniflex Guidance |

| **Measurement Details** | |
|---|---|
| Measurement type: | Single scan |
| Sample mode: | Reflection |

| *Scan* | |
|---|---|
| Scan range: | 3.000 - 40.000 ° (2θ) |
| Step size: | 0.01 ° (2θ) |
| Speed: | 10.0 °/min (2θ) |
| Scan mode: | Continuous |

| *Used wavelength* | |
|---|---|
| Intended wavelength type: | Kα1 |
| Kα1: | 1.540598 Å |
| Kα2: | 1.544426 Å |
| Kα2/Kα1 intensity ratio: | 0.50 |
| Kα: | 1.541874 Å |
| Kα: | 1.392250 Å |

| **Instrument Details** | |
|---|---|
| *X-Ray Generator* | |
| Tube output voltage: | 40 kV |
| Tube output: | 15 mA |
| High-voltage generation method: | High-frequency Cockcroft-Walton method |
| Stability: | Within ±0.05% for both the tube voltage and tube current, with reference to ±10% of input power variation |

| *X-ray tube* | |
|---|---|
| Name: | Toshiba Analix type A-26L |
| Anode material: | Cu |
| Maximus output: | 0.60 kW |
| Focus size: | 1 × 10 mm |

| *Kβ Filter* | |
|---|---|
| Name: | Ni-filter |
| Thickness (mm): | 0.015 |
| Material: | Ni |

| *Goniometer (Angle measuring device)* | |
|---|---|
| Type: | Vertical θ/2θ |
| Goniometer radius: | 150 mm |
| Scanning axis: | θ/2θ linked |
| 2θ scanning range: | +2 ° to +140 ° |
| θ/2θ axis minimum step angle: | 0.005 ° (2θ) |
| Position speed: | 500 °/min (2θ) |
| Scanning speed: | 0.01 to 100 °/min |
| Datum angle: | 2θ = 10 ° |
| X-ray take-off angle: | 6 ° (fixed) |

| *Slit* | |
|---|---|
| DS: | 1.25 ° |
| IHS: | 10.0 mm |
| SS: | none (open) |
| RS: | none (open) |
| Incident side Soller slit: | 2.5 ° |
| Receiving side Soller slit: | 2.5 ° |

| *Detector* | |
|---|---|
| Name: | D/teX Ultra High-speed 1D Detector |
| Detection element: | 1D semiconductor element |
| Window material: | Be |
| Effective window size: | 13 mm (H) × 20 mm (W) |
| Dimensions: | 80 mm (L) |

### Thermal Analysis

DSC analysis was carried out using a DSC Mettler Toledo DSC1.

The sample was weighed in an aluminum pan hermetically sealed with an aluminum cover. The analysis was performed heating the sample from 25°C to 320°C at 10K/min.

TG analysis was carried out using the Mettler Toledo TGA/DSC1.

The sample was weighed in an aluminum pan hermetically sealed with an aluminum pierced cover. The analysis was performed heating the sample from 25°C to 320°C at 10K/min

### Example 1. Preparation and Characterization of Crystalline Form 1 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide (HM06) Free Base

### Example 1A: Preparation of (HM06 free base Form 1)

HM06 free base Form 1 was prepared using a Sonogashira cross-coupling reaction mediated by Pd(PPh₃)₂Cl₂ and CuI in ACN. More specifically, 4-amino-6-bromo-N-(4-(methoxymethyl)phenyl)-7-(1-methylcyclopropyl)-6,7-dihydro-5*H*-pyrrolo[2,3-*d*]pyrimidine-5-carboxamide (8.2Kg) (which can be prepared according to known methods, such as Example 55 of U.S. Patent No. 10,155,768) was added to a reaction vessel at room temperature and the following reagents were added: CuI (0.108Kg), Pd(PPh₃)₂Cl₂ (0.402Kg) and CH₃CN (82.5 L). 4-(prop-2-yn-1-yl)morpholine (6.683Kg) was added to the mass. TEA (7.9 L) was added. The mass was inerted with vacuum and N₂ five times (500 mbar/1030 mbar). The mass was heated to Tj 60°C (Ti: 56°C) under N₂ and it was stirred for 14 hours at Tj 60°C to obtain a solution, and then cooled to Ti 18-22°C. THF (82.2 L) was added and the mixture was warmed to Ti 40°C. The warmed mixture was filtered through a 8-10 µm mesh filter (PTFE) by applying just pressure (at least 2 bars). The filtered mix was cooled to Ti 20-25°C. The mixture was then passed through a plug of resin (ISOLUTE^{®} Si-Thiol; Ti 25-25°C mix by applying RATE: 220 L/h). The reaction vessel, the filter, and the resin cake were washed with THF (13 L). The wet resin was discarded. The solvent was distilled at Tj: 45°C under vacuum until applying Vmax (by stopping stirring when needed). MeTHF (164.5 L) was added to the residue and stirred at room temperature to obtain a homogeneous suspension.

To the organic suspension 0.1 M solution of N-acetylcysteine (2.6 Kg) in water (161.6 L) was added. The mass was heated to Tj: 50°C (Ti: 45-48°C) and stirred for 3 hours. The stirring was then stopped, and the phases were allowed to separate for at least 20 minutes. After phases separation, the aqueous layer was back-extracted with MeTHF (81.9 L) at Ti 45-48°C. The mixture was stirred for 30 minutes and then the stirring was stopped, and the layers were allowed to separate for at least 20 minutes at Ti:45-48°C. The layers were then separated, and the aqueous layer was disposed of. The two organic layers were combined and then NaCl 20% (41 L) was added at Ti:45-48°C. The mixture was stirred for 30 minutes and then stirring was stopped, and the phases were allowed to separate for at least 20 minutes. The aqueous layer was removed and disposed of. Water (66.4 L) was added to the organic layer at Ti:45-48°C. The mixture was stirred for 30 minutes at the same temperature and then stirring was stopped and the phases were allowed to separate for at least 20 minutes. The aqueous layer was again removed and discarded.

The organic layer was distilled to residue at Tj: 45°C under vacuum until applying Vmax. The residue was stripped overnight at Tj: 45°C under Vmax without stirring. Acetone was added (17 L) to the residue at Tj: 45°C and then heated to Ti: 48°C (Tj: 52°C). The mixture was stirred for at least 1 hour to obtain a homogeneous suspension. The suspension was cooled to Ti: -10°C (Tj: -15°C) over at least 3 hours. The product was then isolated by filtration using a 20 um mesh filter at Tj: -10°C by applying vacuum and pressure (at least 2 bars). The filter cake was washed with pre-cooled acetone (3.6 L; Ti: -10°C) by applying pressure (at least 2 bars) and vacuum until no more deliquoring was observed. The solid was dried at Tj: 60°C for at least 24 hours to obtain the final product (6.8 Kg). The product was stored at Tj: 2-8°C.

**Figure 1A** shows an XRPD pattern of HM06 crystalline free base Form 1 obtained using CuKα radiation. Peaks identified in Figure 1A include those set forth in Table 2:

**Table 2**

| **Pos. [°2θ]** | **Rel. Int. [%]** | **d-spacing [Å]** | **Height [cts]** |
|---|---|---|---|
| 12.57 | 48.89 | 7.04 | 2921.13 |
| 13.36 | 93.72 | 6.63 | 5600.08 |
| 16.08 | 27.05 | 5.51 | 1616.5 |
| 18.86 | 72.5 | 4.71 | 4332.07 |
| 20.66 | 53.94 | 4.30 | 3223.03 |
| 21.73 | 100 | 4.09 | 5975.17 |
| 23.90 | 61.89 | 3.72 | 3698.17 |
| 24.86 | 85.18 | 3.58 | 5089.73 |

### Example 1B: Preparation and Characterization of HM06 Free Base Crystalline Forms 2-5 and an HCl Salt Crystalline Form A

Five (5) new crystalline phases for the free base, and an HCl crystalline salt form, were observed after selected re-crystallization experiments using the solvents listed in the Table 3.

**Table 3**

| **Polymorph label** | **Re-crystallization procedure** |
|---|---|
| Form 1 | Starting material |
| Form 2 | Evaporation at RT from MET |
| Form 3 | Evaporation at RT from ACT |
| Form 4 | Evaporation at RT from DCM |
| Form 5 | Evaporation at RT from THF |
| Form A | Slurry in water at RT (with HCl 1: 1) |

**Figure 1B** shows an XRPD pattern of HM06 crystalline free base Form 2 obtained using CuKα radiation. Peaks identified in Figure 1B include those set forth in Table 4:

**Table 4**

| **Pos. [°2θ]** | **Rel. Int. [%]** | **d-spacing [Å]** | **Height [cts]** |
|---|---|---|---|
| 7.94 | 100 | 11.13 | 27925.95 |
| 10.47 | 10.48 | 8.45 | 2925.98 |
| 11.53 | 3.22 | 7.68 | 900.48 |
| 15.75 | 24.48 | 5.63 | 6836.33 |
| 21.80 | 27.79 | 4.08 | 7759.4 |
| 23.65 | 21.72 | 3.76 | 6065.91 |

**Figure 1C** shows an XRPD pattern of HM06 crystalline free base Form 3 obtained using CuKα radiation. Peaks identified in Figure 1C include those set forth in Table 5:

**Table 5**

| **Pos. [°2θ]** | **Rel. Int. [%]** | **d-spacing [Å]** | **Height [cts]** |
|---|---|---|---|
| 7.11 | 100 | 12.43 | 10316.09 |
| 7.83 | 47.45 | 11.28 | 4895.41 |
| 14.12 | 31.74 | 6.27 | 3274.2 |
| 16.15 | 19.65 | 5.49 | 2027.61 |
| 20.61 | 70.5 | 4.31 | 7272.47 |
| 21.19 | 15.76 | 4.19 | 1626.32 |
| 26.37 | 16.07 | 3.38 | 1657.95 |
| 28.59 | 11.13 | 3.12 | 1148.26 |

**Figure 1D** shows an XRPD pattern of HM06 crystalline free base Form 4 obtained using CuKα radiation. Peaks identified in Figure 1D include those set forth in Table 6:

**Table 6**

| **Pos. [°2θ]** | **Rel. Int. [%]** | **d-spacing [Å]** | **Height [cts]** |
|---|---|---|---|
| 7.77 | 41.27 | 11.37 | 1862.12 |
| 9.48 | 46.72 | 9.33 | 2108.04 |
| 11.54 | 48.29 | 7.67 | 2179.18 |
| 16.34 | 44.97 | 5.42 | 2029.31 |
| 20.21 | 31.92 | 4.39 | 1440.3 |
| 23.24 | 100 | 3.83 | 4512.34 |
| 24.77 | 37.42 | 3.59 | 1688.46 |

**Figure 1E** shows an XRPD pattern of HM06 crystalline free base Form 5 obtained using CuKα radiation. Peaks identified in Figure 1E include those set forth in Table 7:

**Table 7**

| **Pos. [°2θ]** | **Rel. Int. [%]** | **d-spacing [Å]** | **Height [cts]** |
|---|---|---|---|
| 9.51 | 100 | 9.30 | 12313.21 |
| 13.52 | 30.35 | 6.55 | 3737.24 |
| 18.71 | 67.06 | 4.74 | 8257.43 |
| 21.26 | 32.42 | 4.18 | 3992.48 |
| 21.49 | 28.77 | 4.14 | 3542.66 |
| 28.60 | 21.18 | 3.12 | 2607.57 |
| 29.05 | 23.61 | 3.07 | 2906.57 |

**Figure 1F** shows an XRPD pattern of an HM06 HCl salt Form A obtained using CuKα radiation. Peaks identified in Figure 1F include those set forth in Table 8:

**Table 8**

| **Pos. [°20]** | **Rel. Int. [%]** | **d-spacing [Å]** | **Height [cts]** |
|---|---|---|---|
| 5.67 | 8.39 | 15.59 | 703.84 |
| 7.19 | 83.68 | 12.29 | 7023.54 |
| 7.32 | 100 | 12.07 | 8393.81 |
| 10.90 | 6.37 | 8.12 | 534.67 |
| 14.31 | 16.97 | 6.19 | 1424.06 |
| 14.59 | 25.75 | 6.07 | 2161.27 |
| 20.08 | 13.06 | 4.42 | 1095.9 |
| 21.24 | 11.38 | 4.18 | 954.99 |

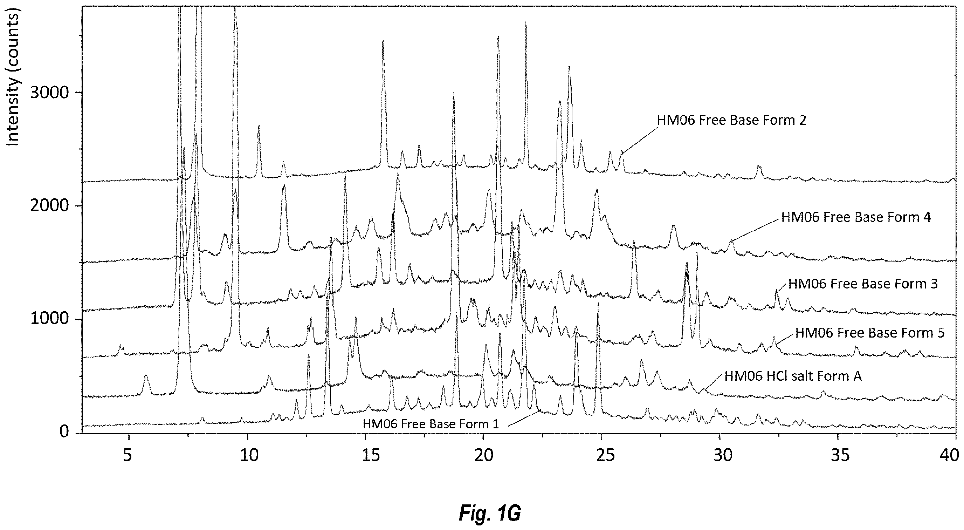

**Figure 1G** shows an overlay of XRPD patterns of five HM06 crystalline free base forms and an HM06 HCl salt Form A obtained using CuKα radiation.

### Example 2. Synthesis and Characterization of Crystalline 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide (HM06) 1:1 HCl salt Form 1

In a 100 ml single-neck round-bottom flask, 1.16 ml (I. l eq.) of HCl solution 2M in water was added to l g of HM06 free base. 40 ml of tetrahydrofuran was charged and the suspension was allowed to stir (800 rpm) at room temperature (25°C) for two days.

A sampling was collected, filtered, and analyzed by XRPD. The remaining suspension was recovered by suction, dried under vacuum (50 mbar) at 25°C for one day. The dried sample was analyzed by XRPD.

**Figure 2A** shows an XRPD pattern of crystalline HM06 1:1 HCl Form 1 obtained using CuKα radiation. **Figures 2B** **and** **2C** also show XRPD patterns of crystalline HM06 1:1 HCl Form 1 obtained using CuKα radiation. Peaks identified in **Figures 2A-2C** for crystalline HM06 1:1 HCl include those set forth in Table 9:

**Table 9**

| **Pos. [°2Th.]** | **Rel. Int. [%]** | **d-spacing [Å]** | **Height [cts]** |
|---|---|---|---|
| 6.53 | 100 | 13.54 | 1304.09 |
| 7.37 | 55.63 | 11.99 | 725.45 |
| 9.07 | 19.8 | 9.75 | 258.24 |
| 14.60 | 41.77 | 6.07 | 544.69 |
| 16.35 | 55.13 | 5.42 | 718.92 |
| 21.26 | 40.68 | 4.18 | 530.47 |
| 26.12 | 74.38 | 3.41 | 969.95 |

### Thermal Analysis

The DSC profile of HM06 1:1 HCl Form 1 recorded in a sealed pan showed an event after 200°C ascribable to sample melt and degradation. **Figure 2D** is a DSC thermogram of HM06 1:1 HCl Form 1. The lack of the signal in the DSC profile related to solvent release and the change in the baseline was probably due to the sealed pan used combined with the effect due to solvent release.

The TGA profile of HM06 1:1 HCl Form 1 showed a weight loss of 4.8% in the range of 40°-170°C consistent with water evolution as recorded by EGA. Degradation occurred after 200°C. **Figure 2E** provides the TGA profile of HM06 1:1 HCl Form 1. The heat-flow recorded in TGA showed a broad and large event that took place in the range of water loss and a signal after 200°C ascribable to melt, then degradation occurred.

### Example 3. Preparation of HM06 1:2 HCl

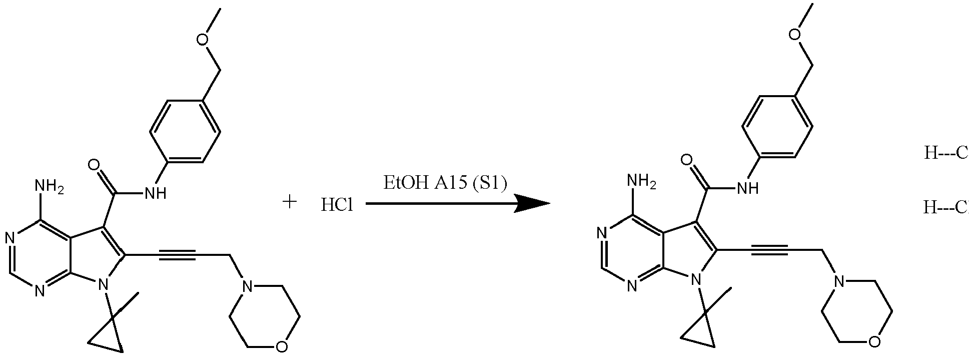

### A. Synthesis of 1G of HM06 1:2 HCl using aqueous HCl

1 g of HM06 free base was weighed and transferred in a 250 mL reactor equipped with a magnetic stirring bar. 50 mL of ethanol were then added and the resulting mixture was heated until complete dissolution of the solid (T = 80 °C). When no solid material was observed, the solution was cooled to 50 °C. 532 µL (3 eq.) of HCl 37% were slowly added into the reactor. The formation of a solid was immediately observed. The mixture was cooled to 25 °C in 25 minutes and then stirred for additional 1 hour. After this time, the formed solid was isolated by vacuum filtration, washed with ethanol and dried at 40 °C and 30 mbar for 24 hours. 1.08 g of product was recovered as white solid in nearly quantitative yield.

### B. Synthesis of 5G of HM06 1:2 HCl using aqueous HCl

5 g of HM06 free base were weighed and transferred in a 250 mL reactor equipped with a magnetic stirring bar. 70 mL of ethanol were then added and the resulting mixture was heated until complete dissolution of the solid (T = 80 °C). When no solid material was observed, the solution was cooled at 50 °C. Precipitation of a small amount of solid was observed, so the solution was heated again until complete dissolution and then cooled to 60°C. At this temperature, no formation of precipitate was observed. 2.5 mL (3 eq.) of HCl 37% were then dissolved in 10 mL of ethanol and the obtained solution was slowly added in the reactor. The formation of a solid was immediately observed. The mixture was cooled at 25 °C in 35 minutes, and then stirred for additional 1 hour. After this time, the formed solid was isolated by vacuum filtration, washed with ethanol and dried at 40 °C and 30 mbar for 24 hours. 5.64 g of product was recovered as a white solid in nearly quantitative yield.

### C. Synthesis of 2.5G of HM06 1:2 HCl using anhydrous HCl

2.5 g of HM06 free base were weighed and transferred into a 250 mL reactor equipped with a magnetic stirring bar. 35 mL of ethanol were then added and the resulting mixture was heated until complete dissolution of the solid (T = 80 °C). When no solid material was observed, the solution was cooled at 50 °C. 4.8 mL (3 eq.) of anhydrous HCl 3.3 M in ethanol were mixed with 5 mL of ethanol and the resulting solution was slowly added into the reactor. The formation of a solid was immediately observed. The mixture was cooled to 25 °C in 35 minutes, and then stirred for additional 1 hour. After this time, the formed solid was isolated by vacuum filtration, washed with additional 10 mL of ethanol and then collected and dried at 25 °C and 0.1 mbar for 24 hours. The solid was further dried at 100 °C and 30 mbar for additional 72 hours. TG/EG analysis confirmed the recovery of an anhydrous compound. 2.69 g of product is recovered as a white solid in nearly quantitative yield.

### D. Stoichiometry determination

To determine the stoichiometry of the salt, chloride analysis was performed by ionic chromatography. A solution of HM06 1:2 HCl was prepared by dissolving 149.2 mg of powder in a volumetric flask (10 mL) with water (HPLC grade). Based on the TGA analysis performed on the batch just before the chloride determination, (weight loss associated to water content of 5.9%) the amount of dosed anhydrous salt was considered to be 140.4 mg (94.1%).

Assuming a stoichiometry of 1:2 (HM06:HCl), the molecular weight of the anhydrous salt is 547.5 g/mol that corresponds to a concentration of 0.0256 mmol/mL of HM06 in the prepared solution. The chloride concentration determined by ionic chromatography was found to be 0.05056 mmol/mL corresponding to a chlorides/HM06 molar ratio of 1.98, confirming the HM06:HCl stoichiometry of 1:2.

### Example 4. Preparation and Characterization of Polymorphic Forms 1 and Form 1-bis of HM06 1:2 HCl

### Example 4A: Synthesis of HM06 1:2 HCl Form 1

HM06 free base (562.0 g) was added to hot ethanol (7885.5 mL) and the mass was heated at Ti: 75°C with stirring until the solid completely dissolved. The solution then underwent polishing filtration over a 1 µm cartridge (PP or PTFE). A mixture of HCl 33% (283.4 mL) and EtOH (283.5 mL) was added over at least 1 hr to the pre-filtered solution with stirring while maintaining a temperature of Ti: 65-75°C (target 70°C). The mass was then cooled to Ti: 20-25°C over at least 30 min and then stirred at Ti: 20-25°C for at least 1 hour. The mixture was then isolated by filtration on a 20 um mesh filter by applying pressure (at least 2 bars) and vacuum until no more deliquoring was observed. The filter cake was washed twice with EtOH (1070.8 mL × 2) by applying pressure (at least 2 bars) and vacuum until no more deliquoring was observed. The wet product was dried at Tj: 40°C for at least 12 hours. The product HM06 1:2 HCl Form 1 was obtained (626 g). The product was stored at Tj: 2-8°C.

**Figure 3A** shows an XRPD pattern the crystalline HM06 1:2 HCl Form 1 obtained using CuKα radiation. Peaks identified in **Figure 3A** include those set forth in Table 10:

**Table 10**

| **Pos. [°2θ]** | **Rel. Int. [%]** | **d-spacing [Å]** | **Height [cts]** |
|---|---|---|---|
| 6.59 | 28.15 | 13.39 | 698.32 |
| 7.40 | 13.52 | 11.93 | 335.24 |
| 9.12 | 8.08 | 9.69 | 200.37 |
| 14.57 | 19.97 | 6.08 | 495.45 |
| 16.39 | 16.88 | 5.40 | 418.62 |
| 26.06 | 100 | 3.42 | 2480.39 |
| 26.57 | 23.49 | 3.35 | 582.53 |
| 27.07 | 16.43 | 3.29 | 407.42 |

Thermal analysis was performed after 2 months of storage at low temperature (4-10°C) in a sealed container. **Figure 3B** provides the TGA profile of HM06 1:2 HCl Form 1. A weight loss of 4.5% was observed in the range 30-160°C ascribable to water release as confirmed by EGA. Above approx. 200°C, degradation took place. TGA showed a further weight loss ascribable to methanol release as confirmed by EGA. **Figure 3C** is a DSC thermogram of HM06 1:2 HCl Form 1. As shown in the figure, the DSC showed a broad endothermic event ascribable to water release starting at 25°C and lasting up to approx. 160°C. The following endothermic peak at 195.13°C (onset 188.11°C) was associated to sample melting.

### Single Crystal of HM06 1:2 HCl Form 1

Crystals of HM06 1:2 HCl Form 1 were obtained by slow evaporation. The crystals were big enough for single crystal diffraction, but all were affected by non-merohedry twinning, which means that two crystals grow together to form the same macroscopic sample. It was not possible to separate the two crystals and collected data clearly showed the presence of two reciprocal lattices (see **Figure 3D**). The solution and refinement of the structure was influenced by this situation.

Two data sets were collected of two different crystals. Both cases were twinned crystals and the second lattice was obtained by the rotation of 180° along b* of the first lattice. In the first case, the crystal was made of two almost equal components, and the non-merohedry twinning affected badly the data and did not allow one to refine the structure to obtain good R values. The second data collection was characterized by one dominant component and a second weaker component. In this case, the refinement was acceptable.

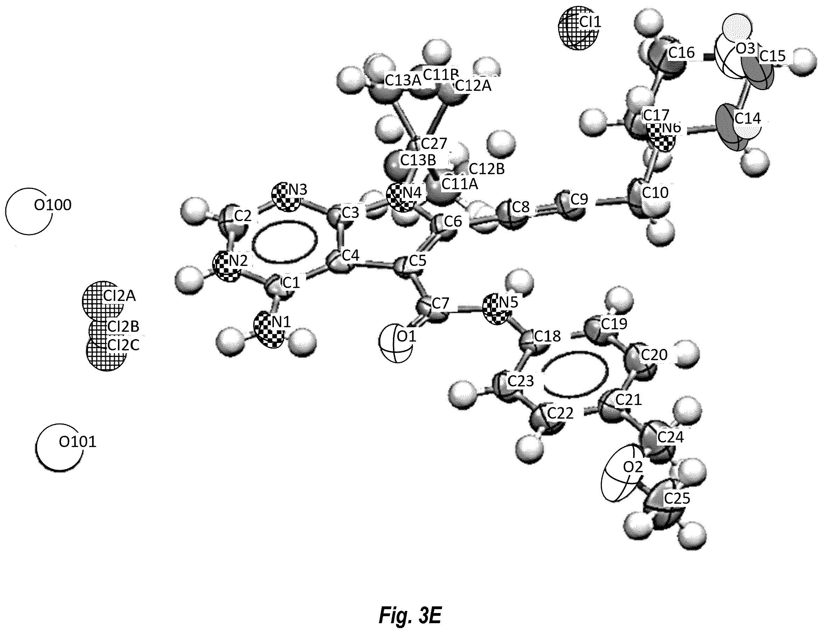

The HM06 1:2HCl Form 1 crystallizes as monoclinic in space group P2₁\c and parameters a= 6.8636(8) Å, b= 16.7683(12) Å, c= 24.5798(13) Å , β= 94.163(7)° and V= 2821.4(4)³. The asymmetric unit consists of one HM06 diprotonated, two chloride ions, and 1.35 water molecules located in two position (see **Figure 3E**). The chloride ion labelled Cl2 is disordered over three positions with occupancy 0.35, 0.36 and 0.29 respecitvley for Cl2A, Cl2B, Cl2C. Probably the position of the Cl2 depends on the number of water molecules in the cell, since the Cl⁻ and the oxygen in the water molecule may be repelling each other.

The HM06 molecules form columns along the b axis which present short contacts (molecules distance of 3.4Å) ascribible to the presence of π-stack interactions. The columns have a kind of cross section, which propbably prevents collapse of the structure upon the removal of water molecules (see **Error! Reference source not found.4A and 4B**).

**Table 11 - Crystal data and structure refinement for HM06 1:2 HCl Form 1**

| **PARAMETER** | **RESULT** | |
|---|---|---|
| Identification code | Shelx | |
| Empirical formula | C26 H35 Cl2 N6 O4.35 | |
| Formula weight | 572.1 | |
| Temperature | 293(2) K | |
| Wavelength | 0.71073 Å | |
| Crystal system | Monoclinic | |
| Space group | P 21/c | |
| Unit cell dimensions | a = 6.8636(8) Å | α = 90°. |
| | b = 16.7683(12) Å | β = 94.163(7)°. |
| | c = 24.5798(13) Å | γ = 90°. |
| | | |
| Volume | 2821.4(4) Å³ | |
| Z | 4 | |
| Density (calculated) | 1.352 Mg/m³ | |
| Absorption coefficient | 0.275 mm⁻¹ | |
| F(000) | 1212 | |
| Crystal size | 0.220 × 0.140 × 0.020 mm³ | |
| Theta range for data collection | 3.302 to 29.097°. | |
| Index ranges | -5<=h<=8, -22<=k<=21, -32<=l<=32 | |
| Reflections collected | 9501 | |
| Independent reflections | 5288 [R(int) = 0.0959] | |
| Completeness to theta = 25.000° | 84.2 % | |
| Refinement method | Full-matrix least-squares on F² | |
| Data / restraints / parameters | 5288/1/347 | |
| Goodness-of-fit on F² | 1.065 | |
| Final R indices [I>2sigma(I)] | R1 = 0.1529, wR2 = 0.3200 | |
| R indices (all data) | R1 = 0.3078, wR2 = 0.3980 | |
| Extinction coefficient | n/a | |
| Largest diff. peak and hole | 0.736 and -0.723 e.Å⁻³ | |

### Example 4B: HM06 1:2 HCl Form 1-bis

To determine the hydrate/anhydrous nature of HM06 1:2 HCl Form 1, and to define the exact amount of water present in the crystal lattice, a set of preliminary dehydration/drying experiments were performed, which led to the discovery of Form 1-bis.

**Figure 5A** shows an XRPD pattern of crystalline HM06 1:2 HCl Form 1-bis obtained using CuKα radiation. Peaks identified in Figure 5A include those set forth in Table 12:

**Table 12**

| **Pos. [°2θ]** | **Rel. Int. [%]** | **d-spacing [Å]** | **Height [cts]** |
|---|---|---|---|
| 6.66 | 81.8 | 13.28 | 1584.99 |
| 7.63 | 45.13 | 11.59 | 874.4 |
| 9.31 | 24.47 | 9.50 | 474.17 |
| 10.74 | 61.19 | 8.24 | 1185.61 |
| 13.09 | 59.39 | 6.76 | 1150.79 |
| 16.45 | 98.83 | 5.39 | 1914.82 |
| 21.36 | 100 | 4.16 | 1937.55 |
| 26.70 | 74.65 | 3.34 | 1446.44 |
| 29.01 | 63.65 | 3.08 | 1233.28 |

VP-XRPD measurements were performed on the Panalytical X'pert equipped with the Anton Paar TTK450 chamber which allowed for measurement of the powder in-situ at controlled temperature and/or under vacuum.

The first measurement was collected at RT and atmospheric pressure. The sample was then left for 15 minutes under vacuum (0.07 mbar). **Figure 5B** shows an XRPD pattern of HM06 1:2 HCl crystalline Form 1-bis obtained using CuKα radiation. As shown in Figure 5B, the second pattern labelled Form 1-bis is different with respect to the pattern of the starting material (Form 1) since the vacuum leads to the dehydration of the sample.

It can be noted that some peaks do not change their position, while others clearly shift to higher theta values; probably the release of the water molecules affects some crystallographic planes while the structure does not dramatically change. Based on the structure determination by SC-XRD, Form 1-bis was supposed to be a very unstable anhydrous form not drastically different from Form 1. This behavior allows the easy uptake of the water molecule in short time.

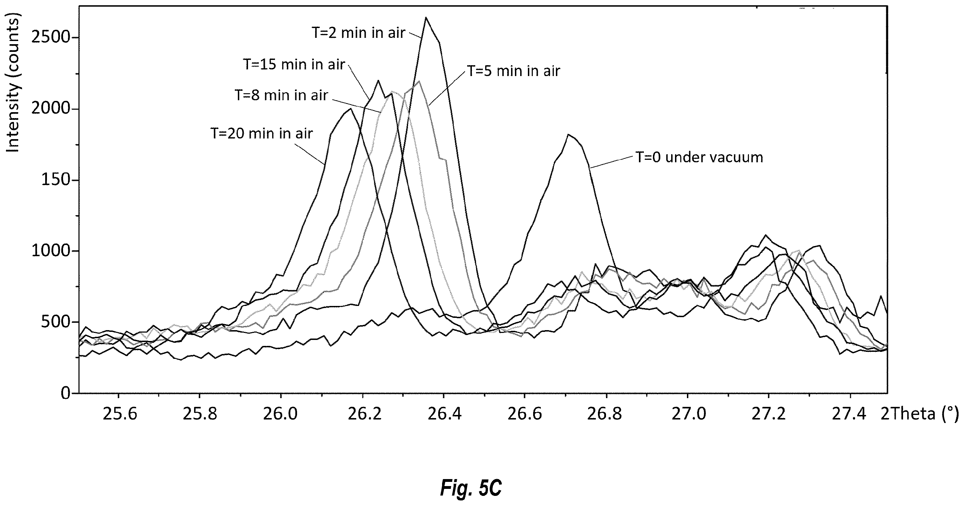

The uptake of water by Form 1-bis was observed via XRPD by following the difference in the pattern in the range 2θ= 25.5°- 27-5° (see **Figure 5C**). Under vacuum the highest peak is the peak at 2θ = 26.7° while after exposure of the sample in air 2 minutes the highest is at 2θ= 26.4°. The peak at 2θ = 26.4° moves to 2θ= 26.2° in 20 minutes. HM06 1:2 HCl crystalline Form 1-bis uptakes the water from the atmosphere as soon as the powder is exposed to the air, and it reachs the diffraction pattern of the starting material in 20 minutes (see Error! Reference source not found.**5D**). This experiment was done in ambient condition with RH% of the room about at 80%.

### Example 5. Synthesis and Characterization of Crystalline HM06 1:2 HCl Form 2

HM06 1:2 HCl Form 2 was observed in mixture with Form 1 from a high temperature (50°C) slurry experiment using ethanol. 15 mg of HM06 1:2 HCl Form 1 was suspended in 1.5 mL of ethanol and allowed to stir at 50°C for three days. After this time, the suspension was filtered under vacuum under approx. 45-50 %RH and analyzed by XRPD. Its diffraction pattern is reported in **Figure 6A** as the top pattern, compared with XRPD patterns for Form 1 and a standard pattern for Form 2 (bottom two lines).

### Reproduction and micro scale-up procedures

### 1. Reproduction procedure

The crystallization procedure was reproduced twice. Reproduction R01 lead to HM06 1:2 HCl Form 2 affected with minor traces of Form 3, while pure Form 2 was recovered from reproduction R02. For both the experiments, the filtration step and the preparation of the plate for the XRPD analysis were conducted under 7% RH. The XRPD measurement was performed using a Kapton film. A summary of the obtained results and the relative XRPD patterns are reported in Table 13.

**Table 13 Example 5 Reproduction procedures results.**

| **Crystallization Procedure** | **Reproduction** | **Result** |
|---|---|---|
| 3-days slurry experiments from Ethanol at 50°C | R00 | Mixture of Form 1 + Form 2 |
| 3-days slurry experiments from Ethanol at 50°C¹ | R01 | Form 2 + minor traces of Form 3 |
| 3-days slurry experiments from Ethanol at 50°C¹ | R02 | Form 2 |

| | | |
|---|---|---|
| ¹The isolation of the powder by vacuum filtration and the preparation of the sample plate covered with Kapton film was performed at 7% RH. | | |

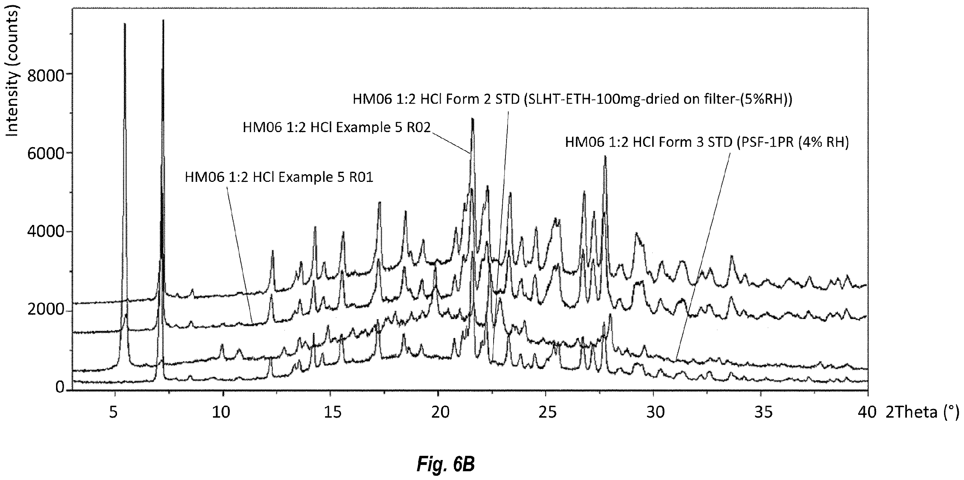

The XRPD patterns of the results of R01 and R02, as compared to standard reference patterns of Form 2 and Form 3, are shown in **Figure 6B****.**

### Micro scale-up procedure

Different micro scale-up procedures were attempted to obtain enough powder to be used for further tests and to probe the process feasibility. The first trial was carried out on 100 mg of HM06 1:2 HCl Form 1. The powder was suspended in 10 mL of ethanol (10mg/mL) and allowed to stir at 50°C for four days. After this time, the suspension was filtered under vacuum under 5% RH conditions and the preparation of the XRPD plate covered with Kapton film was conducted under the same % RH conditions. HM06 1:2 HCl Form 2 was isolated and the collected XRPD pattern was used as standard (STD) reference pattern of Form 2.

This procedure was reproduced twice, and the isolation step performed after five days under 5% RH conditions. The first reproduction (R01) resulted in HM06 1:2 HCl Form 3 affected by some traces of Form 2, while the second reproduction (R02) resulted in Form 3 with one signal at 7.2° 2Theta, which was ascribable to Form 2.

Taking into account this data, a further procedure was attempted. 100 mg of HM06 1:2 HCl Form 1 was suspended in 5 mL of ethanol (20mg/mL) and left to stir at 50°C for ten days. From this procedure, Form 2 was isolated with a small signal at 5.5°2 theta from Form 3.

A summary of the obtained results are reported in Table 14.

**Table 14 Example 5 Micro scale-up procedure results.**

| **Crystallization Procedure** | **Reproduction** | **Result** |
|---|---|---|
| 4-days slurry experiments from Ethanol at 50°C | R00 | Form 2 (STD) |
| 5-days slurry experiments from Ethanol at 50°C | R01 | Form 3 + traces of Form 2 |
| 5-days slurry experiments from Ethanol at 50°C | R02 | Form 3 + minor signal at 7.2° 2theta ascribable to Form 2 |
| 10-days slurry experiments from Ethanol at 50°C¹ | R00 | Form 2 + minor signal at 5.5° 2theta ascribable to Form 3 |

| | | |
|---|---|---|
| ¹Experiment performed using a concentration of 20mg/mL. | | |

**Figure 6C** shows the XRPD pattern of HM06 1:2 HCl Form 2 collected after 4-days slurry experiments from ethanol scaled on 100 mg obtained using CuKα radiation, and used as STD reference. Peaks identified in Figure 6C include those set forth in Table 15:

**Table 15**

| **Pos. [°2θ]** | **Rel. Int. [%]** | **d-spacing [Å]** | **Height [cts]** |
|---|---|---|---|
| | | | |
| 7.13 | 100 | 12.40 | 3787.19 |
| 12.21 | 13.31 | 7.25 | 504.23 |
| 14.22 | 22.89 | 6.23 | 866.79 |
| 15.50 | 18.78 | 5.72 | 711.14 |
| 17.18 | 26.16 | 5.16 | 990.59 |
| 21.60 | 71.72 | 4.11 | 2716.08 |
| 22.23 | 32.09 | 4.00 | 1215.17 |
| 23.26 | 19.33 | 3.82 | 732.11 |
| 26.72 | 21.58 | 3.34 | 817.23 |
| 27.69 | 31.62 | 3.22 | 1197.42 |

### Thermal Analysis

**Figure 6D** is a DSC thermogram of HM06 1:1 HCl Form 2, which showed an endothermic event at 219.8°C (onset at 205.5°C) consistent with sample melting. Above approx. 200°C, degradation took place.

**Figure 6E** provides the TGA profile of HM06 1:1 HCl Form 2, which showed no weight loss. The sample can be considered anhydrous. The methanol and HCl release was detected during degradation.

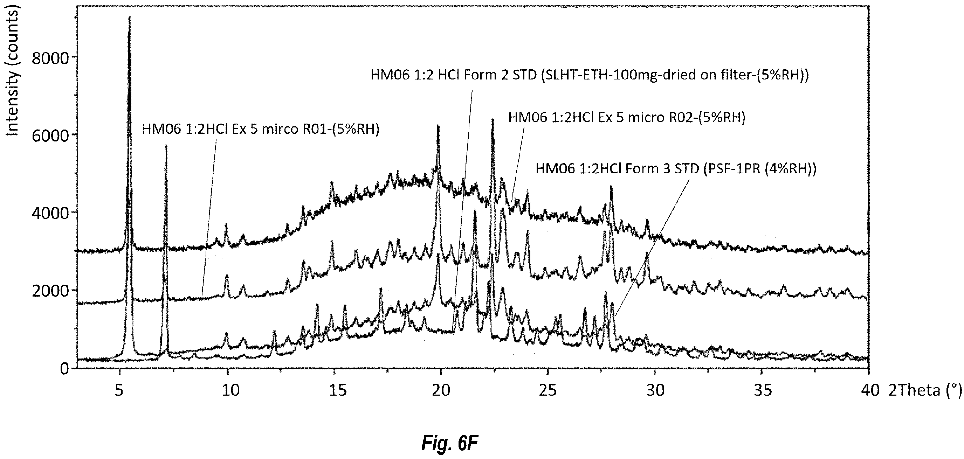

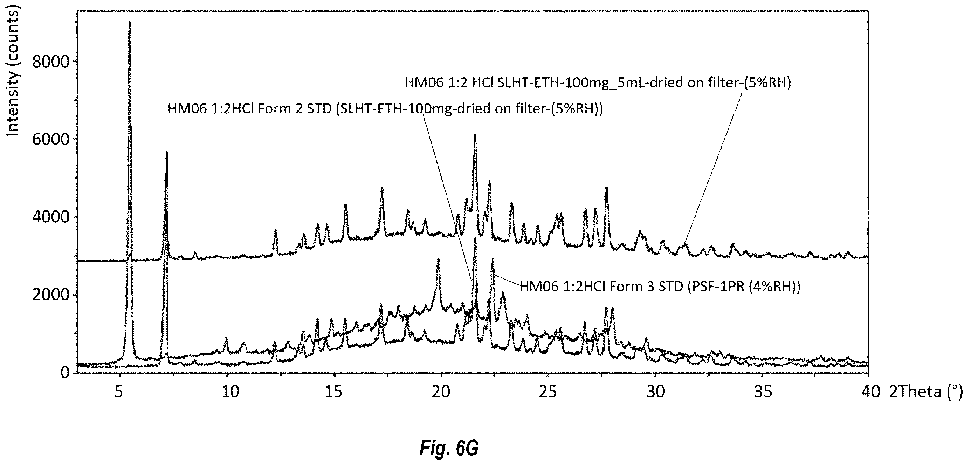

**Figures 6F** **and** **6G** show the relative XRPD patterns from the Example 5 micro scale ups obtained using CuKα radiation.

### Example 6. Synthesis and Characterization of Form 3 of HM06 1:2 HCl

15 mg of HM06 1:2 HCl Form 1 was suspended in 1.5 mL of acetonitrile and it was allowed to stir at 50°C degree for three days. After this time, the suspension was filtered under vacuum under approx. 45-50 %RH and analyzed by XRPD. **Figure 7A** shows an XRPD pattern overlay of crystalline HM06 1:2 HCl Form 3 obtained after HT (50°C) slurry experiments from acetonitrile (top line), and the standard reference patterns of Form 1 (black line,) Form 2 (bottom line), and Form 3 (pink line) obtained using CuKα radiation.

### Reproduction and micro scale-up procedures

### Reproduction procedure

The crystallization procedure was reproduced twice, extending the time up to nine days and treating the recovered powder at 7% RH condition. Kapton film was used to prepare the XRPD plate. Reproduction R01 led to a phase in which some traces of Form 2 were observed. Reproduction R02 lead to Form 3 and some further unassigned peaks.

Since pure Form 3 was collected from a fast precipitation experiment using 1-propanol and used as the standard reference pattern, additional reproductions were attempted. All three fast gradient trials were prepared as follows. To 15 mg of HM06 1:2 HCl Form 1 was added 1.5 mL of 1-propanol. The suspension was heated up to the solvent's boiling point for few minutes. A clear solution was immediately observed. It was crash cooled to 10°C using an ice bath. The precipitation occurred immediately. The powder was recovered by under vacuum filtration and the XRPD plate was prepared using a Kapton film. All these experiments were treated under controlled 4-5% RH conditions.

A summary of the obtained results are reported in Table 16.

**Table 16 Example 6 Reproduction procedures results.**

| **Crystallization Procedure** | **Reproduction** | **Result** |
|---|---|---|
| 3-days slurry experiments from Acetonitrile at 50°C | R00 | Form 1 + Form 3 with minor traces of Form 2 |
| 9-days slurry experiments from Acetonitrile at 50°C¹ | R01 | Form 3² + traces of Form 2 and further unassigned peaks |
| 9-days slurry experiments from Acetonitrile at 50°C¹ | R02 | Form 3² + further unassigned peaks |
| Fast gradient precipitation from 1-Propanol³ | R00 | Form 3 (STD) |
| Fast gradient precipitation from 1-Propanol³ | R01 | Form 3+ -Form 2+ further unassigned peaks. |
| Fast gradient precipitation from 1-Propanol³ | R02 | Form 3+ -Form 2+ further unassigned peaks. |

| | | |
|---|---|---|
| ¹The isolation of the powder by vacuum filtration and the preparation of the sample plate covered with Kapton film was performed at 7% RH. ²The intensity of the signal at 5.5° 2theta showed to be lower if compared with the standard reference pattern of Form 3. ³The isolation of the powder by vacuum filtration and the preparation of the sample plate covered with Kapton film was performed at 4-5% RH. | | |

### Micro scale-up procedure

Different micro scale-up procedures were attempted to obtain enough powder to be used for further tests and to probe the process feasibility. All the trials were treated under controlled %RH conditions between 5-7% values both for the isolation step and the preparation of the XRPD sample plate for which a Kapton film was used.

The first trial was carried out on 100 mg of HM06 1:2 HCl Form 1. The powder was suspended in 10 mL of acetonitrile (10mg/mL) and allowed to stir at 50°C for four days. After this time, the suspension was filtered under vacuum and analyzed by XRPD. A mixture of Form 2 and Form 3 was recovered. To try to reach pure Form 3, a first reproduction R01 was planned, extending the slurry time up to twelve days. In parallel, the same experiment tested on a concentration of 20 mg/mL was also prepared. From reproduction R01, Form 2 was gathered, while the other trial led to a mixture of Form 2 and Form 3 with the observation of an unassigned peak at 6.3° 2theta.

Since pure Form 3 was achieved from a fast gradient precipitation from 1-propanol performed on 15 mg and in spite of the reproductions did not lead to Form 3, a micro scale-up procedure was pursued. 100 mg of HM06 1:2 HCl Form 1 was suspended in 10 mL of 1-propanol. It was heated up to the solvent's boiling point. After few minutes, the obtained clear solution was crash cooled at 10°C and left under magnetic stirring for 5 minutes. Then the powder was isolated by filtration and analyzed by XRPD. Pure Form 3 was achieved and its XRPD pattern was used as standard reference. A very minor signal at 7.2° 2theta, probably attributable to Form 2, was observed. The filtration step and the preparation of the XRPD sample plate covered with Kapton film was performed at 4% RH.

Reproduction R01 was conducted following the procedure noted in Table 17 and a drying process was applied. A sampling was analyzed by XRPD. Form 3 was attained so the entire wet cake was treated at 40°C/50 mbar for three hours and then re-measured. Form 3 was achieved although a minor signal at 7.2° 2theta ascribable to Form 2 was detected.

Two further reproductions (R02 and R03) were carried out according to the same procedure. Form 3 was collected although a minor signal at 7.2° 2theta ascribable to Form 2 was detected. No additional drying step was applied for R03. Reproduction R02 was instead subjected to two dying steps followed both by TGA-EGA analysis. The low crystallinity degree showed by R02 was due to the low amount used for the XRPD analysis.

Based on these results, fast gradient precipitation from 1-propanol can be considered as a suitable procedure to obtain Form 3.

A summary of the obtained results is reported in Table 17.

**Table 17 Example 6 Micro scale-up procedure results.**

| **Crystallization Procedure** | **Reproduction** | **Result** |
|---|---|---|
| 4-days slurry experiments from Acetonitrile at 50°C | R00 | Form 2 and Form 3 |
| 12-days slurry experiments from Acetonitrile at 50°C | R01 | Form 2 |
| 12-days slurry experiments from Acetonitrile at 50°C¹ | R00 | Form 2 + traces of Form 3 + signal at 6.3° 2theta |
| Fast gradient precipitation from 1-Propanol² | R00 | Form 3 |
| Fast gradient precipitation from 1-Propanol² | R01 | Form 3 + minor signal of Form 2 |
| Fast gradient precipitation from 1-Propanol² | R02 | Form 3 + minor signal of Form 2 |
| Fast gradient precipitation from 1-Propanol² | R03 | Form 3 + minor signal of Form 2 |

| | | |
|---|---|---|
| ¹Experiment achieved using a concentration of 20mg/mL. ²The isolation of the powder by vacuum filtration and the preparation of the sample plate covered with Kapton film was performed between 3-7% RH. | | |

**Figure 7B** shows an XRPD pattern of HM06 1:2 HCl Form 3 collected after fast gradient precipitation from 1-propanol scaled on 100 mg obtained using CuKα radiation. Peaks from HM06 1:2 HCl Form 3 identified in Figure 7B include those set forth in Table 17:

**Table 17**

| **Pos. [°2θ]** | **Rel. Int. [%]** | **d-spacing [Å]** | **Height [cts]** |
|---|---|---|---|
| | | | |
| 5.44 | 100 | 16.24 | 9465.65 |
| 9.94 | 2.61 | 8.89 | 246.62 |
| 14.85 | 2.52 | 5.96 | 238.46 |
| 22.39 | 11.18 | 3.97 | 1058.73 |
| 22.84 | 5.63 | 3.89 | 532.59 |
| 27.96 | 6.41 | 3.19 | 606.82 |

**Figure 7C** is a DSC thermogram of HM06 1:2 HCl Form 3, which showed an endothermic event at 214°C (onset at 202°C) ascribable to sample melting. Above approx. 200°C, degradation took place.

**Figure 7D** provides the TGA profile of HM06 1:2 HCl Form 3, which showed a very mild weight loss of 0.7% up to 180°C. During degradation methanol and HCl evolution was detected by EGA.

### Example 7. Synthesis and Characterization of Form 4 and Form 4-bis of HM06 1:2 HCl

### Form 04 and Form 04-bis Crystallization procedure

Form 4-bis was collected after evaporation experiment from methanol at 25°C under low pressure according to the following procedure:

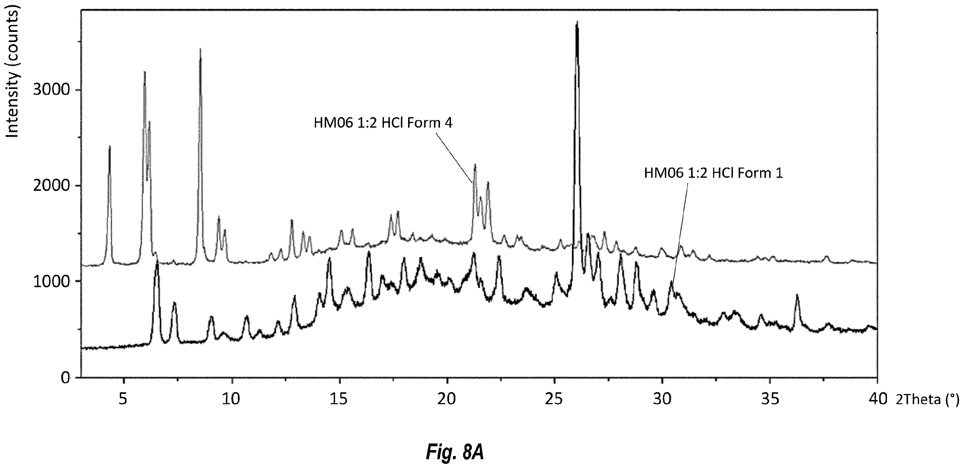

A saturated solution of HM06 1:2 HCl Form 1 in methanol approx. 50mg/mL was prepared and allowed to stir at room temperature overnight (18 hours). After this time, it was filtered and left to evaporate at 25°C/700 mbar. The preparation of the sample plate sealed with Kapton film was performed under 40-45% RH. The collected XRPD pattern compared with Form 1 is reported in **Figure 8A****.** Some very minor signals of Form 1 were present.

### Reproduction procedure

The experiment was reproduced four times following the procedure reported above (reproductions R01-R04). The preparation of the XRPD sample plate sealed with Kapton film was performed under 40-45% RH. In all the trials, Form 1 was recovered. This procedure was attempted another four times, but in this case the sample was isolated under controlled % RH with values between 7-8% RH. In all the analyzed samples, a new pattern labelled as Form 4, affected by signals of Form 1, was observed. From a qualitative point of view, reproduction R05 showed the lowest amount of Form 1. Table 18 sets forth the reproduction procedures and results.

**Table 18: Example 7 Reproduction procedures results.**

| **Crystallization Procedure** | **Reproduction** | **Result** |
|---|---|---|
| Low pressure room temperature evaporation experiment from Methanol saturated solution | R01 | Form 1 |
| Low pressure room temperature evaporation experiment from Methanol saturated solution | R02 | Form 1 |
| Low pressure room temperature evaporation experiment from Methanol saturated solution | R03 | Form 1 |
| Low pressure room temperature evaporation experiment from Methanol saturated solution | R04 | Form 1 |
| Low pressure room temperature evaporation experiment from Methanol saturated solution¹ | R05 | Form 4 + Form 1 |
| Low pressure room temperature evaporation experiment from Methanol saturated solution¹ | R06 | Form 4 + Form 1 |
| Low pressure room temperature evaporation experiment from Methanol saturated solution¹ | R07 | Form 4 + Form 1 |
| Low pressure room temperature evaporation experiment from Methanol saturated solution¹ | R08 | Form 4 + Form 1 |

| | | |
|---|---|---|
| ¹The isolation of the powder by vacuum filtration and the preparation of the sample plate covered with Kapton film was performed at 7-8% RH. | | |

**Figure 8B** shows an XRPD pattern of HM06 1:2 HCl Form 4-bis. Peaks identified in Figure 8B for HM06 1:2 HCl Form 4-bis include those set forth in Table 19:

**Table 19**

| **Pos. [°2θ]** | **Rel. Int. [%]** | **d-spacing [Å]** | **Height [cts]** |
|---|---|---|---|
| | | | |
| 4.35 | 53.07 | 20.31158 | 2097.32 |
| 5.98 | 86.96 | 14.78 | 3436.58 |
| 6.20 | 62.02 | 14.26 | 2451.18 |
| 8.54 | 100 | 10.34 | 3952.05 |
| 17.39 | 12.98 | 5.10 | 512.89 |
| 21.28 | 36.31 | 4.17 | 1434.97 |
| 21.58 | 21.35 | 4.12 | 843.79 |
| 21.89 | 28.67 | 4.06 | 1133.05 |

### Stability assessment

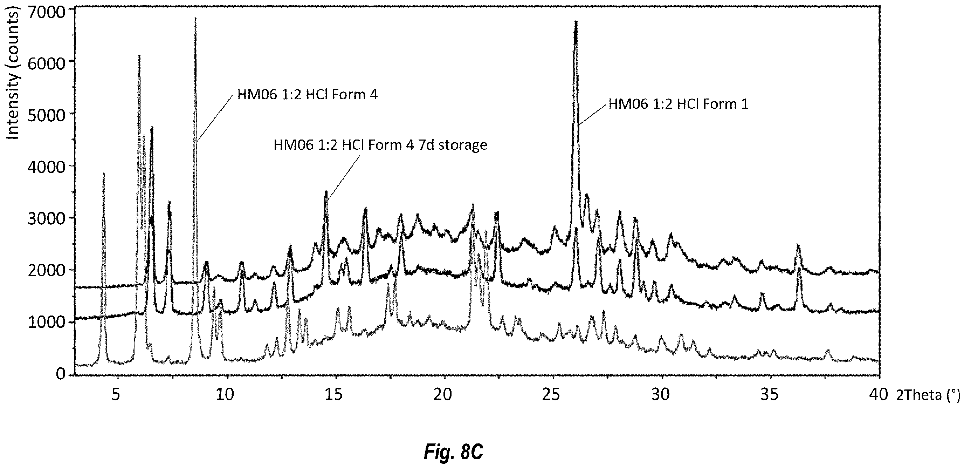

The stability of Form 4-bis was assessed after seven days of storage in a sealed vial. It showed a complete conversion into Form 1 as shown in **Figure 8C****.**

### Form 4 Crystallization procedure

As described in the previous Section, HM06 1:2 HCl Form 4 was isolated in mixture with Form 1 from the reproduction experiments that were performed in an attempt to achieve Form 4-bis.

### Reproduction procedure

As described in the previous Section and in Table 18, Form 4 was recovered from all the four reproduction experiments performed (R05-R08). Reproduction R05 seemed to have the lowest amount of Form 1 from a qualitative point of view.

**Figure 8D** shows an XRPD pattern of HM06 1:2 HCl Form 4 obtained using CuKα radiation. Peaks identified in Figure 8D of HM06 1:2 HCl Form 4 include those set forth in Table 20:

**Table 20**

| **Pos. [°2θ]** | **Rel. Int. [%]** | **d-spacing [Å]** | **Height [cts]** |
|---|---|---|---|
| | | | |
| 4.38 | 50.26 | 20.17 | 1626.57 |
| 6.15 | 100 | 14.38 | 3236.01 |
| 8.60 | 56.6 | 10.28 | 1831.54 |
| 9.62 | 25.53 | 9.20 | 826.04 |
| 21.46 | 23.82 | 4.14 | 770.9 |
| 21.90 | 31 | 4.06 | 1003.06 |
| 26.14 | 19.45 | 3.41 | 629.35 |

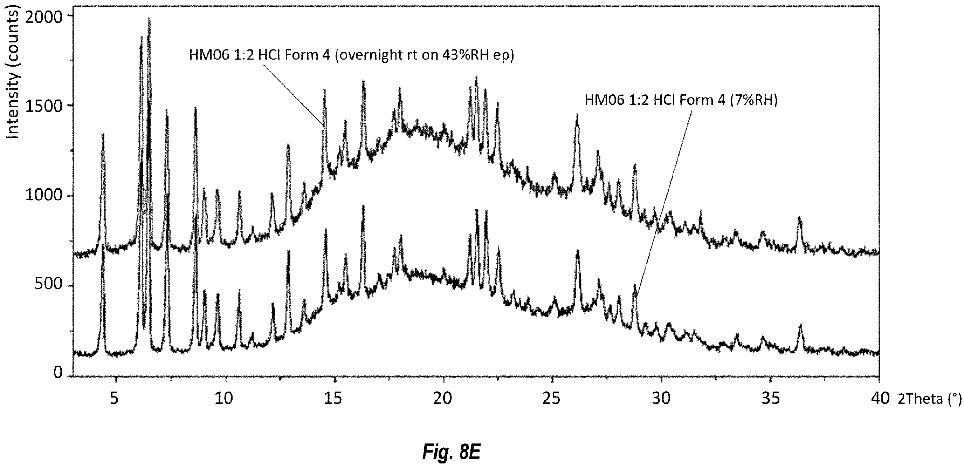

HM06 1:2 HCl Form 4 exposed powder was stored overnight at room temperature under 43% RH. No significant modifications were observed as shown in **Figure 8E****.**

### Example 8. Synthesis and Characterization of Form 5 and Form 5-bis of HM06 1:2 HCl

### Form 5 and Form 5-bis

### Form 5-bis

### 1. Crystallization procedure

An evaporation experiment of HM06 1:2 HCl Form 1 in a mixture of 50/50 water/dimethylformamide at 60°C led to the isolation of a new XRPD pattern in which some signals from Form 5 were observed. Because of the similarity, it was labelled as Form 5-bis. The treatment of the sample was performed under 40-45% RH condition.

**Figure 9A** shows the XRPD pattern of HM06 1:2 HCl Form 5-bis compared with Form 5.

### 2. Reproduction procedure

The evaporation experiment was reproduced twice and an orange-colored powder was collected. Both samples were analyzed by XRPD and presented the diffraction pattern of Form 5, with some minor signals probably associated with Form 5-bis and further unassigned, in particular one at 18° 2theta with a discrete intensity. Based on these results Form 5-bis was considered as not reproducible.

**Figure 9B** shows an XRPD pattern of HM06 1:2 HCl Form 5-bis. Peaks identified in Figure 9B for HM06 1:2 HCl Form 5-bis include those set forth in Table 21:

**Table 21**

| **Pos. [°2θ]** | **Rel. Int. [%]** | **d-spacing [Å]** | **Height [cts]** |
|---|---|---|---|
| | | | |
| 6.08 | 40.98 | 14.52 | 197.22 |
| 6.82 | 50.02 | 12.95 | 240.75 |
| 7.11 | 42.35 | 12.41 | 203.82 |
| 7.51 | 34.6 | 11.77 | 166.5 |
| 8.92 | 34.51 | 9.90 | 166.08 |
| 9.35 | 31.35 | 9.45 | 150.86 |
| 11.34 | 54.64 | 7.79 | 262.98 |
| 17.29 | 56.19 | 5.13 | 270.43 |
| 20.02 | 100 | 4.43 | 481.26 |
| 21.21 | 56.52 | 4.18 | 271.99 |
| 22.36 | 33 | 3.97 | 158.83 |
| 23.15 | 92.74 | 3.84 | 446.3 |

### 3. Stability test

The stability of the HM06 1:2 HCl Form 5-bis sample was assessed after 18 hours exposed to air and after one week in a sealed vial, both at room temperature.

Form 5-bis was measured after 18 hours exposed powder at room temperature. The %RH was approx. of 45%. Its XRPD pattern displayed some modifications: e.g., lack of signals at 6° and 12° 2theta and the rising of a peak at 6.4° 2theta.

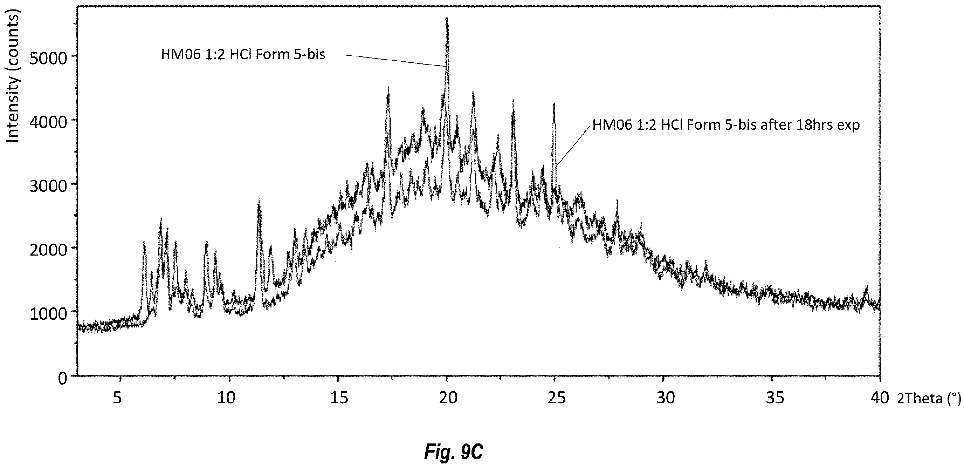

These changes could not be associated with conversion into one of the other isolated polymorphs observed during this study: thus, the sample was considered not stable. **Figure 9C** shows the XRPD pattern of Form 5-bis (blue top pattern) and the same sample analyzed after 18 hrs exposed (bottom red pattern).

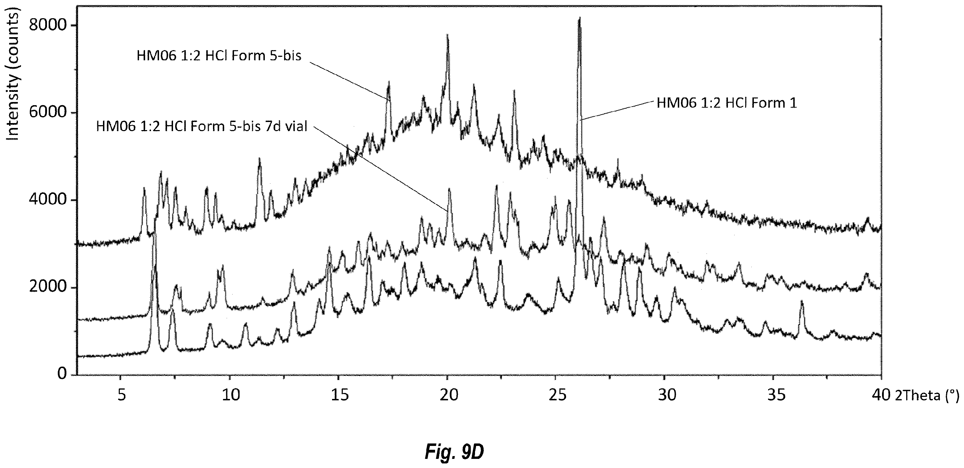

HM06 1:2 HCl Form 5-bis was measured after seven days at room temperature in a sealed vial. As shown in **Figure 9D****,** the sample started to convert into Form 1.

### Form 5 Crystallization procedure/Reproduction procedure

An evaporation experiment of HM06 1:2HCl Form 1 in dimethyl sulfoxide at 60°C gave Form 1 with further unassigned signals. To better understand the nature of these few new signals, the re-crystallization procedure was reproduced twice: R01 and R02. Both samples showed a dark brown color and while R02 was completely vitreous, few powders from R01 were able to be recovered. The powders from R01 were analyzed by XRPD and showed a diffraction pattern with a low crystallinity degree that was labelled as Form 5.

**Figure 9E** shows the XRPD pattern of HM06 1:2 HCl Form 5 obtained using CuKα radiation. Peaks identified in Figure 9E for HM06 1:2 HCl Form 5 include those set forth in Table 22:

**Table 22**

| **Pos. [°2θ]** | **Rel. Int. [%]** | **d-spacing [Å]** | **Height [cts]** |
|---|---|---|---|
| | | | |
| 6.74 | 100 | 13.12 | 385.23 |
| 7.11 | 87.12 | 12.43 | 335.59 |
| 8.10 | 51.92 | 10.91 | 200 |
| 13.10 | 60.05 | 6.76 | 231.33 |
| 17.16 | 46.08 | 5.17 | 177.5 |
| 23.28 | 51.41 | 3.82 | 198.06 |
| 24.22 | 37.3 | 3.67 | 143.68 |
| 25.15 | 27.09 | 3.54 | 104.35 |
| 26.24 | 45.49 | 3.40 | 175.23 |

**Figure 9F** is a DSC thermogram of HM06 1:2 HCl Form 5, which showed an endothermic broad event between 30°C-90°C ascribable to solvent release as was also observed in TGA-EGA. Two consecutive endothermic events at 158.7°C (onset at 144.6°C) and 164.8°C (onset at 158.3°C) were also observed.

**Figure 9G** provides the TGA profile of HM06 1:2 HCl Form 5, which showed a weight loss of water up to 130°C. It was not possible to clearly ascribe the evolution of water to dehydration or adsorbed water release. Above 200°C, degradation took place. The EGA did not detect the HCl evolution as observed for the other isolated forms. The formation of a salt with a lower HCl content might not be excluded; the stoichiometry of the salt in Form 5 was not determined definitively.

### Example 9. Synthesis and Characterization of Form 6 of HM06 1:2 HCl

Form 6 was collected after evaporation of HM06 1:2 HCl Form 1 in a 1:1 acetonitrile/water solution at room temperature under low pressure. After 6 days of storage under these conditions, conversion into Form 1 was observed. Since the batch showed an orange color and because of its phase instability, no further analysis was performed.

**Figure 10** shows an XRPD pattern of HM06 1:2 HCl Form 6 using CuKα radiation. Peaks identified in Figure 10 for HM06 1:2 HCl Form 6 include those set forth in Table 23:

**Table 23**

| **Pos. [°2θ]** | **Rel. Int. [%]** | **d-spacing [Å]** | **Height [cts]** |
|---|---|---|---|
| | | | |
| 5.88 | 85.66 | 15.04 | 624.28 |
| 7.01 | 61.26 | 12.61 | 446.47 |
| 8.81 | 47.9 | 10.04 | 349.12 |
| 11.51 | 80.31 | 7.69 | 585.32 |
| 13.12 | 61.1 | 6.75 | 445.33 |
| 18.36 | 79.24 | 4.83 | 577.52 |
| 21.4 | 59.23 | 4.15 | 431.7 |
| 22.92 | 100 | 3.88 | 728.8 |

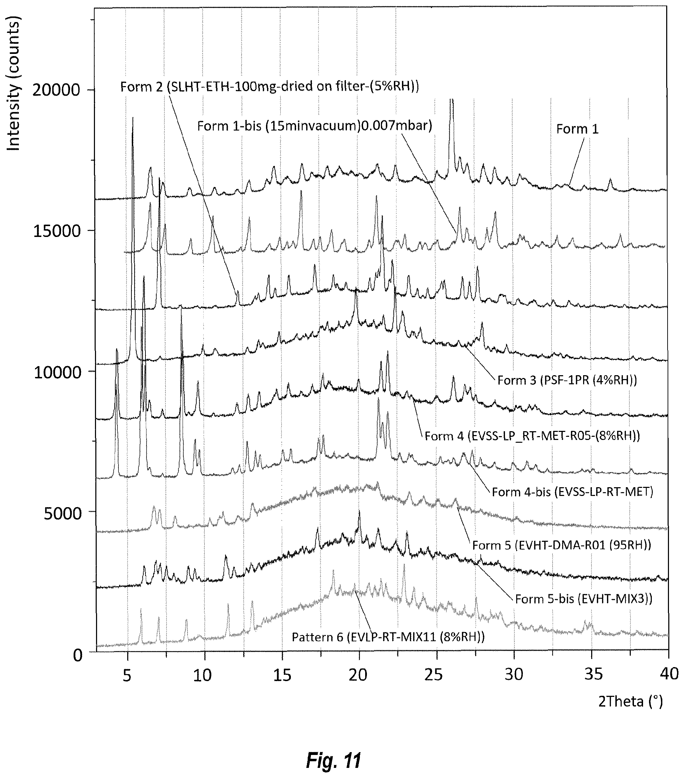

**Figure 11** reports an overlay of XRPD patterns of all isolated forms of HM06 1:2 HCl.

### Example 10: Storage Stability Testing for HM06 1:2 HCl Form 1

Samples of HM06 1:2 HCl Form 1 were subjected to accelerated storage conditions and long-term storage. The results demonstrated that HM06 1:2 HCl Form 1 remained stable and crystalline.

### 1. Accelerated Storage Conditions

Samples of HM06 1:2 HCl Form 1 from the same lot were stored at 40°C and 75% relative humidity for 24 months with testing at regular intervals. Table 24 sets forth the sample analysis at times 0, 1 month, 3 months, and 6 months.

**Table 24: Accelerated conditions - 40°C / 75% relative humidity**

| **Test** | ***Acceptance Criteria*** | **Time Point (months)** | | | |
|---|---|---|---|---|---|
| | | **0** | **1** | **3** | **6** |
| Appearance | *N*/*A* | Light brown solid | Light brown solid | Light brown solid | Off-white solid |
| Water content (KF) | *max. 8%* | 6% | 6% | 5% | 5% |
| HPLC purity:* | | | | | |
| - 12-HM06 | *min. 96%* | 99% | 99% | 99% | 99% |
| - 12-HM06.il** | *N*/*A* | 0.51% | 0.51% | 0.51% | 0.51% |
| - RRT 0.56 | *N*/*A* | not detected | not detected | not detected | 0.05% |
| - RRT 1.11 | *N*/*A* | not detected | not detected | not detected | 0.05% |
| - RRT 1.20-1.24 | *N*/*A* | 0.20% | 0.20% | 0.20% | 0.19% |
| - RRT 1.41-1.42 | *N*/*A* | 0.06% | 0.06% | 0.06% | 0.06% |
| HPLC assay (anhydrous) | *95 - 105%* | 99% | 100% | 102% | 98% |
| XRPD | *Report result* | Crystalline | Crystalline | Crystalline | Crystalline |

| | | | | | |
|---|---|---|---|---|---|
| **Report all impurities >0.05% (A%). **RRT (Relative retention time) 0.92-.94* | | | | | |

### 2. 24 Month Storage

Samples of HM06 1:2 HCl Form 1 from the same lot were stored at 25°C and 60% relative humidity for six months with testing at regular intervals. Table 25 sets forth the sample analysis at times 0, 3 months, 6 months, 9 months, 12 months, 18 months, and 24 months.

**Table 25: Long term conditions - 25°C and 60% relative humidity**

| **Test** | ***Acceptance Criteria*** | **Time Point (months)** | | | | | | |
|---|---|---|---|---|---|---|---|---|
| | | **0** | **3** | **6** | **9** | **12** | **18** | **24** |
| Appearance | *N*/*A* | Light brown solid | Light brown solid | Off-white solid | Off-white solid | Off-white solid | Light brown solid | Light brown solid |
| Water content (KF) | *max. 8%* | 6% | 5% | 5% | 5% | 6% | 5% | 6% |
| HPLC purity:* | | | | | | | | |
| - 12-HM06 | *min. 96%* | 99% | 99% | 99% | 99% | 99% | 99% | 99% |
| - RRT 0.85 | *N*/*A* | not detected | not detected | not detected | not detected | not detected | 0.05% | 0.06% |
| - 12-HM06.il** | *N*/*A* | 0.51% | 0.51% | 0.51% | 0.51% | 0.51% | 0.51% | 0.51% |
| -RRT 1.11 | *N*/*A* | not detected | not detected | not detected | not detected | not detected | 0.05% | n.d. |
| - RRT 1.20-1.24 | *N*/*A* | 0.20% | 0.20% | 0.20% | 0.20% | 0.20% | 0.19% | 0.19% |
| - RRT 1.41-1.42 | *N*/*A* | 0.06% | 0.06% | 0.06% | 0.06% | 0.06% | 0.06% | 0.06% |
| HPLC assay (anhydrous) | *95 - 105%* | 99% | 102% | 99% | 99% | 99% | 97% | 100% |
| XRPD | *Report result* | Crystalline | Crystalline | Crystalline | Crystalline | Crystalline | Crystalline | Crystalline |

| | | | | | | | | |
|---|---|---|---|---|---|---|---|---|
| **Report all impurities* >*0.05% (A%);* ** *RRT 0.92-0.94* | | | | | | | | |

The foregoing written specification is considered to be sufficient to enable one skilled in the art to practice the embodiments. The foregoing description and Examples detail certain embodiments and describes the best mode contemplated by the inventors. It will be appreciated, however, that no matter how detailed the foregoing may appear in text, the embodiment may be practiced in many ways and should be construed in accordance with the appended claims and any equivalents thereof.

As used herein, the term about refers to a numeric value, including, for example, whole numbers, fractions, and percentages, whether or not explicitly indicated. The term about generally refers to a range of numerical values (e.g., +/-5-10% of the recited range) that one of ordinary skill in the art would consider equivalent to the recited value (e.g., having the same function or result). When terms such as at least and about precede a list of numerical values or ranges, the terms modify all of the values or ranges provided in the list. In some instances, the term about may include numerical values that are rounded to the nearest significant figure.

## Claims

1. 4-amino-N-[4-(methoxymethyl)phenyl]-7-(1-methylcyclopropyl)-6-(3-morpholinoprop-1-yn-1-yl)-7H-pyrrolo[2,3-d]pyrimidine-5-carboxamide 1:2 HCl salt, in crystalline form 1, **characterized by** an XRPD pattern comprising peaks at about 6.59°2θ, 7.40°2θ, 9.12°2θ, 14.57°2θ, 16.39°2θ, 26.06°2θ, 26.57°2θ, and 27.07°2θ.

2. The product of claim 1, **characterized by** an XRPD pattern the same as Figure 3A.

3. The product of any one of claims 1 to 2, having at least one characteristic chosen from a DSC thermogram the same as Figure 3C and a TGA profile the same as Figure 3B.

4. The product of any one of claims 1 to 3, being at least 50% crystalline form, such as at least 60%, at least 70%, at least 80%, at least 90%, at least 95%, at least 96%, at least 97%, at least 98%, at least 99%, or 100% crystalline.

5. A pharmaceutical composition comprising the crystalline form 1 of any of the preceding claims and a pharmaceutically acceptable excipient.

## Patentansprüche

1. 4-Amino-N-[4-(methoxymethyl)phenyl]-7-(1-methylcyclopropyl)-6-(3-morpholinoprop-1-yn-1-yl)-7H-pyrrolo[2,3-d]pyrimidin-5-carboxamid 1:2 HCl-Salz, in kristalliner Form 1, **gekennzeichnet durch** ein Röntgenpulverdiffraktometrie (XRPD)-Muster umfassend Peaks bei ungefähr 6,59°2θ, 7,40°2θ, 9,12°2θ, 14,57°2θ, 16,39°2θ, 26,06°2θ, 26,57°2θ, und 27,07°2θ.

2. Produkt nach Anspruch 1, **gekennzeichnet durch** ein XRPD-Muster, das dasselbe wie in Figur 3A ist.

3. Produkt nach einem der Ansprüche 1 bis 2, mit mindestens einer Charakteristik ausgewählt auf einen DSC-Thermogramm, das dasselbe wie in Figur 3C und einem TGA-Profil, das dasselbe wie in Figur 3B ist.

4. Produkt nach einem der Ansprüche 1 bis 3, das mindestens 50% in kristalliner Form ist, wie mindestens 60%, mindestens 70%, mindestens 80%, mindestens 90%, mindestens 95%, mindestens 96%, mindestens 97%, mindestens 98%, mindestens 99%, oder 100% kristallin.

5. Pharmazeutische Zusammensetzung umfassend die kristalline Form 1 nach einem der vorstehenden Ansprüche und einen pharmazeutisch verträglichen Hilfsstoff.

## Revendications

1. Sel de 4-amino-N-[4-(méthoxyméthyl)phényl]-7-(1-méthylcyclopropyl)-6-(3-morpholinoprop-1-yn-1-yl)-7H-pyrrolo[2,3-d]pyrimidine-5-carboxamide 1:2 HCl, sous forme cristalline 1, **caractérisé par** un motif de diffraction des rayons X sur poudre, XRPD, comprenant des pics à environ 6,59°2θ, 7,40°2θ, 9,12°2θ, 14,57°2θ, 16,39°2θ, 26,06°2θ, 26,57°2θ, et 27,07°2θ.

2. Produit selon la revendication 1, **caractérisé par** un motif de XRPD qui est le même que la Figure 3A.

3. Produit selon l'une quelconque des revendications 1 à 2, ayant au moins une caractéristique choisie parmi un thermogramme de calorimétrie différentielle à balayage, DSC, qui est le même que la Figure 3C et un profil d'analyse thermogravimétrique, TGA, qui est le même que la Figure 3B.

4. Produit selon l'une quelconque des revendications 1 à 3, qui est sous une forme au moins à 50 % cristalline, telle qu'au moins à 60 %, au moins à 70 %, au moins à 80 %, au moins à 90 %, au moins à 95 %, au moins à 96 %, au moins à 97 %, au moins à 98 %, au moins à 99 %, ou 100 % cristalline.

5. Composition pharmaceutique comprenant la forme cristalline 1 selon l'une quelconque des revendications précédentes et un excipient pharmaceutiquement acceptable.
